# EUROPEAN PATENT SPECIFICATION

(11) **EP 3 458 853 B1**
(45) Date of publication and mention of the grant of the patent: **07.09.2022**
(21) Application number: 17800006.3
(22) Date of filing: 16.05.2017
(51) Int. Cl.: G01N 33/00, G01N 33/487, G01N 33/574

(54) **IMPROVED METHODS FOR MONITORING IMMUNE STATUS OF A SUBJECT**
VERBESSERTE VERFAHREN ZUR ÜBERWACHUNG DES IMMUNSTATUS EINER PERSON
MÉTHODES AMÉLIORÉES POUR SURVEILLER L'ÉTAT IMMUNITAIRE D'UN SUJET

(30) Priority: 16.05.2016 US 201662337263 P
(43) Date of publication of application: 27.03.2019
(73) Proprietor: Onco Tracker, Inc., West Hollywood, CA 90069 (US)
(72) Inventor: BERENSON, James Richard, Beverly Hills, CA 90210 (US)
(74) Representative: Schiweck Weinzierl Koch Patentanwälte Partnerschaft mbB
(86) International application number: PCT/US2017/032894
(87) International publication number: WO 2017/201040

(56) References cited:
- WO-A2-2004/081043
- WO-A2-2006/044582
- US-A1- 2006 136 136
- US-A1- 2016 131 654
- E. SANCHEZ ET AL: "Soluble B-Cell Maturation Antigen Mediates Tumor-Induced Immune Deficiency in Multiple Myeloma", CLINICAL CANCER RESEARCH, vol. 22, no. 13, 9 March 2016 (2016-03-09) , pages 3383-3397, XP055474325, US ISSN: 1078-0432, DOI: 10.1158/1078-0432.CCR-15-2224
- DATABASE MEDLINE [Online] US NATIONAL LIBRARY OF MEDICINE (NLM), BETHESDA, MD, US; March 2016 (2016-03), ABDELGAWAD IMAN A ET AL: "Significance of Some Proliferation Markers and Some Prognostic Factors in Patients with Multiple Myeloma and their Impact on the Patients' Survival.", XP002796649, Database accession no. NLM27268602 & ABDELGAWAD IMAN A ET AL: "Significance of Some Proliferation Markers and Some Prognostic Factors in Patients with Multiple Myeloma and their Impact on the Patients' Survival.", ASIAN PACIFIC JOURNAL OF CANCER PREVENTION : APJCP 2016, vol. 17, no. 5, 2016, pages 2389-2394, ISSN: 2476-762X
- LEMANCEWICZ DOROTA ET AL: "Evaluation of TNF superfamily molecules in multiple myeloma patients: Correlation with biological and clinical features", LEUKEMIA RESEARCH, NEW YORK,NY, US, vol. 37, no. 9, 12 June 2013 (2013-06-12), pages 1089-1093, XP028685567, ISSN: 0145-2126, DOI: 10.1016/J.LEUKRES.2013.05.014
- FRAGIOUDAKI M ET AL: "Serum BAFF levels are related to angiogenesis and prognosis in patients with multiple myeloma", LEUKEMIA RESEARCH, NEW YORK,NY, US, vol. 36, no. 8, 13 March 2012 (2012-03-13) , pages 1004-1008, XP028494960, ISSN: 0145-2126, DOI: 10.1016/J.LEUKRES.2012.03.012 [retrieved on 2012-03-19]
- FRAGIOUDAKI M ET AL: "B cell-activating factor: its clinical significance in multiple myeloma patients", ANNALS OF HEMATOLOGY, SPRINGER, BERLIN, DE, vol. 91, no. 9, 21 April 2012 (2012-04-21) , pages 1413-1418, XP035094156, ISSN: 1432-0584, DOI: 10.1007/S00277-012-1470-X
- MATTHIAS KREUZALER ET AL: "Soluble BAFF Levels Inversely Correlate with Peripheral B Cell Numbers and the Expression of BAFF Receptors", THE JOURNAL OF IMMUNOLOGY, vol. 188, no. 1, 28 November 2011 (2011-11-28), pages 497-503, XP055654814, ISSN: 0022-1767, DOI: 10.4049/jimmunol.1102321
- Suzie Vardanyan: "Serum Levels of B-Cell Maturation Antigen Are Elevated in Waldenström's Macroglobulinemia Patients and Correlate with Disease Status and Conventional M-Protein and IgM Levels | Blood Journal", , 3 December 2015 (2015-12-03), XP055591561, Retrieved from the Internet: URL:http://www.bloodjournal.org/content/12 6/23/1778 [retrieved on 2019-05-23]
- ZHANG PEIPEI ET AL: "B Cell-Activating Factor as a New Potential Marker in Inflammatory Bowel Disease", DIGESTIVE DISEASES AND SCIENCES, SPRINGER NEW YORK LLC, US, vol. 61, no. 9, 7 April 2016 (2016-04-07), pages 2608-2618, XP036027830, ISSN: 0163-2116, DOI: 10.1007/S10620-016-4136-Z [retrieved on 2016-04-07]
- JAVIER CARBONE ET AL: "Potential role of serum BAFF as a biomarker in HIV infection", INFECTIOUS DISEASES, vol. 47, no. 4, 17 February 2015 (2015-02-17), pages 260-262, XP055654807, Abingdon ISSN: 2374-4235, DOI: 10.3109/00365548.2014.1001998

## Description

The Sequence Listing associated with this application is provided electronically.

### BACKGROUND

The technical field of the invention is biomarkers for monitoring multiple myeloma.

The immune system is a system of many biological structures and processes within a subject that protects against disease. To function properly, an immune system must detect a wide variety of agents, known as pathogens, from viruses to parasitic worms, and distinguish them from a subject's own healthy tissue. Therefore, proper functioning of the immune system requires that all components of the immune system work in a coordinated manner to neutralize pathogens. An impairment of a subject's immune system leads to infections by opportunistic pathogens that may eventually prove to be fatal. Conversely, a hyperactive immune system (*e*.*g*., in autoimmune diseases) causes the immune system to attack a subject's normal tissues as if they were foreign organisms. The overall efficiency *(i.e.,* normal, hyperactive, or impaired) of a subject's immune system is referred to as the subject's immune status.

The immune system can be classified into subsystems - innate immunity and adaptive immunity. Defense against pathogens is mediated by the early reactions of innate immunity and the later responses of adaptive immunity. There are two types of adaptive immune responses, called cell-mediated immunity (mediated by T cells) and humoral immunity (mediated by B cells).

B cells or B lymphocytes are a type of white blood cell of the lymphocyte subtype. They function in the humoral immunity component of the adaptive immune system by secreting antibodies. Additionally, B cells present antigens (they are also classified as professional antigen-presenting cells (APCs)) and secrete cytokines. In mammals, B cells mature to plasma cells in the bone marrow.

An important factor in the survival, proliferation, normal development, and maturation of B cells is B-cell activating factor (BAFF). BAFF is a 285-amino-acid type II transmembrane protein that belongs to the superfamily of 19 known Tumor Necrosis Factor (TNF) ligands. This cytokine is expressed in B cell lineage cells, and acts as a potent B cell activator. BAFF has also been shown to play a role in B-cell proliferation and as a specific immunity response enhancer. BAFF is expressed as a membrane-bound type II transmembrane protein on various cell types including monocytes, dendritic cells and bone marrow stromal cells. The transmembrane form of BAFF can be cleaved from the membrane, generating a soluble BAFF polypeptide. Soluble BAFF levels in blood are related closely to the number of circulating B-cells. The homotrimeric soluble form of BAFF binds to and activates the BAFF Receptor (BAFF-R). Signaling through BAFF-R stimulates B lymphocytes to undergo proliferation. (For a review, *see* Rihacek et al., 2015). US2016131654 as prior art for example provides compositions and methods for detecting, diagnosing, prognosing, and monitoring multiple myeloma (MM), chronic lymphocytic leukemia, or B-cell non-Hodgkin lymphoma in a subject using BAFF, wherein the control is the same patient from an earlier time point.

Currently, an objective test to determine the immune status of a subject, specifically monitoring MM, is not available, and the existence of such a disease is determined by a physician's observation of the subject's physical symptoms (*e*.*g*., body temperature or physical discomfort such as pain). However, such observations are subjective and can vary from one physician to another. Furthermore, a rapid and reliable determination of a subject's response to treatment is also currently not available, and would be greatly facilitated by a test that could reliably monitor a subject's immune status at different time points during the course of a treatment regimen. Therefore, there is a need in the art to design a fast, reproducible, inexpensive, and reliable test that can indicate the immune status of a subject, specifically monitor MM of a subject.

The present inventors have now surprisingly found that levels of serum or plasma BAFF polypeptide in a serum or plasma sample of a subject correlate with multiple myeloma in the subject. The present inventors have specifically found that an increased amount of serum or plasma BAFF polypeptide in a serum or plasma sample obtained from a subject compared to BAFF polypeptide in a control sample obtained from a normal healthy subject is indicative of a multiple myeloma (MM) patient who has achieved remission of MM, wherein the BAFF polypeptide comprises an amino acid sequence having at least 75% identity with SEQ ID NO: 1. Such in vitro use of serum or plasma BAFF polypeptide for monitoring MM will be further defined below. Further, it is disclosed herein for illustration purposes only that while a decreased amount of BAFF polypeptide or a fragment thereof in a biological sample obtained from a subject compared to amounts of BAFF polypeptide or a fragment thereof in a biological sample obtained from a normal healthy subject indicates that the subject is suffering from an infection or a disease.

### BRIEF SUMMARY

The present invention relates to an in-vitro use of serum or plasma B-cell activating factor (BAFF) polypeptide for monitoring multiple myeloma of a subject, wherein serum or plasma BAFF polypeptide levels are increased in multiple myeloma patients who have achieved complete remission compared to the levels in healthy control subjects, wherein the BAFF polypeptide comprises an amino acid sequence having at least 75% identity with SEQ ID NO: 1.

Additionally, the present invention relates to the in vitro use as mentioned above, wherein the BAFF polypeptide comprises an amino acid sequence having_at least 80% or at least 90% identity with SEQ ID NO: 1.

Further, the present invention relates to the in vitro use as mentioned above, wherein the BAFF polypeptide is detected using a detection system selected from the group consisting of: an immunohistochemistry, enzyme-linked immunosorbent assay (ELISA), radioimmunoassay (RIA), enzyme immunoassay (EIA), fluorescence immunoassay (FIA), luminescence immunoassay (LIA), lateral flow assay, or strip assay.

The present invention also relates to the in vitro use as mentioned above, wherein the detection is performed using an antibody specific for BAFF polypeptide.

Finally, the present invention also relates to the in vitro use as mentioned above, wherein the antibody specific for BAFF polypeptide is a monoclonal antibody or a polyclonal antibody.

### BRIEF DESCRIPTION OF THE DRAWINGS

The accompanying drawings, which are incorporated in and constitute a part of this specification, illustrate several embodiments of the disclosed method and compositions and together with the description, serve to explain the principles of the disclosed methods.

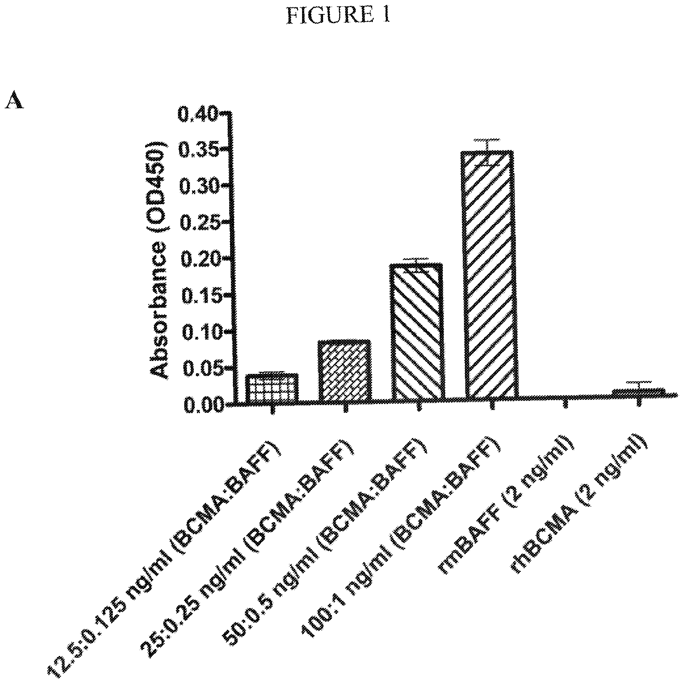

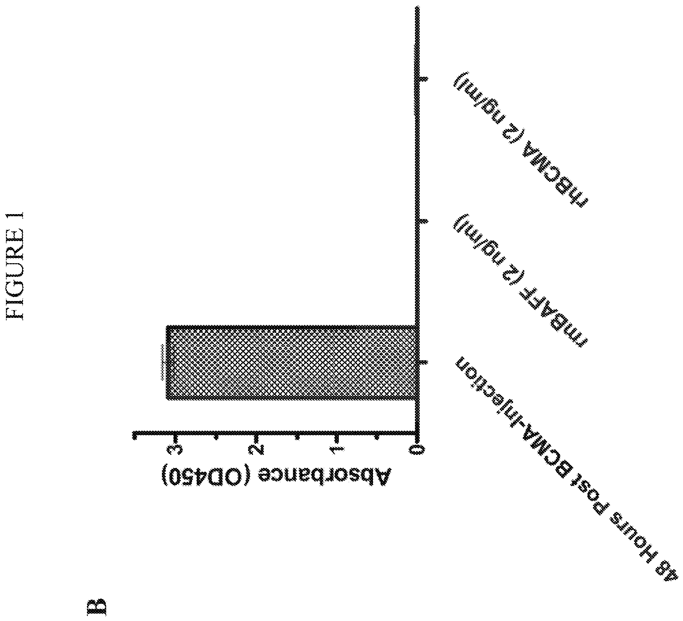

Figure 1 shows that B cell maturation antigen (BCMA) and BAFF form complexes *in vitro.* **A**, Detecting recombinant mouse BAFF (rmBAFF)- recombinant human BCMA (rhBCMA) complexes *in vitro.* **B,** Detecting mouse BAFF (mBAFF; derived from SCID plasma)-rhBCMA complexes *in vitro.*

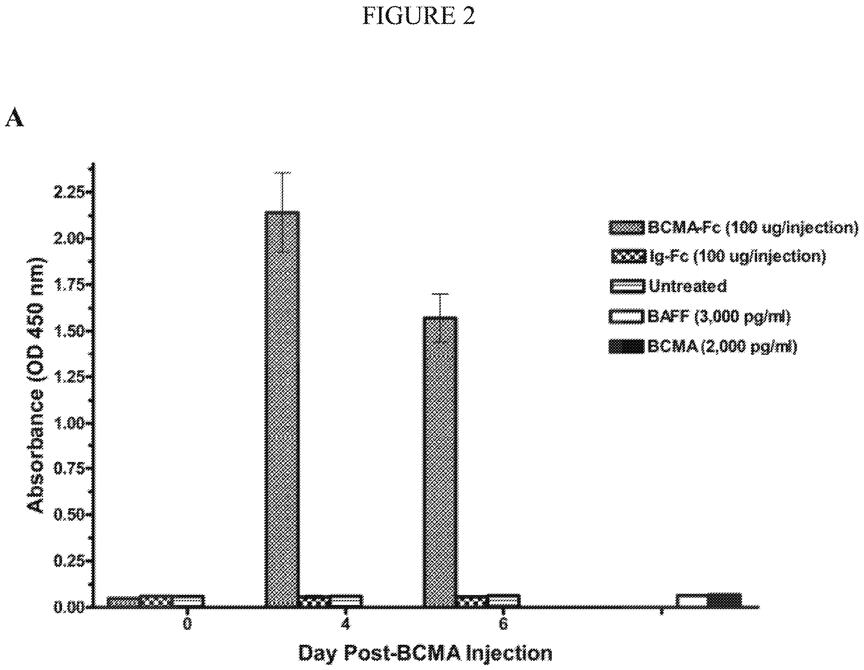

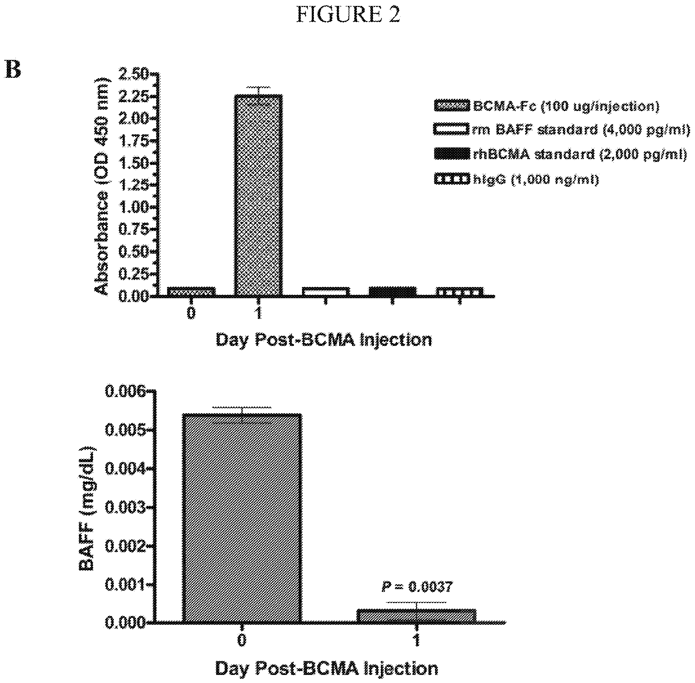

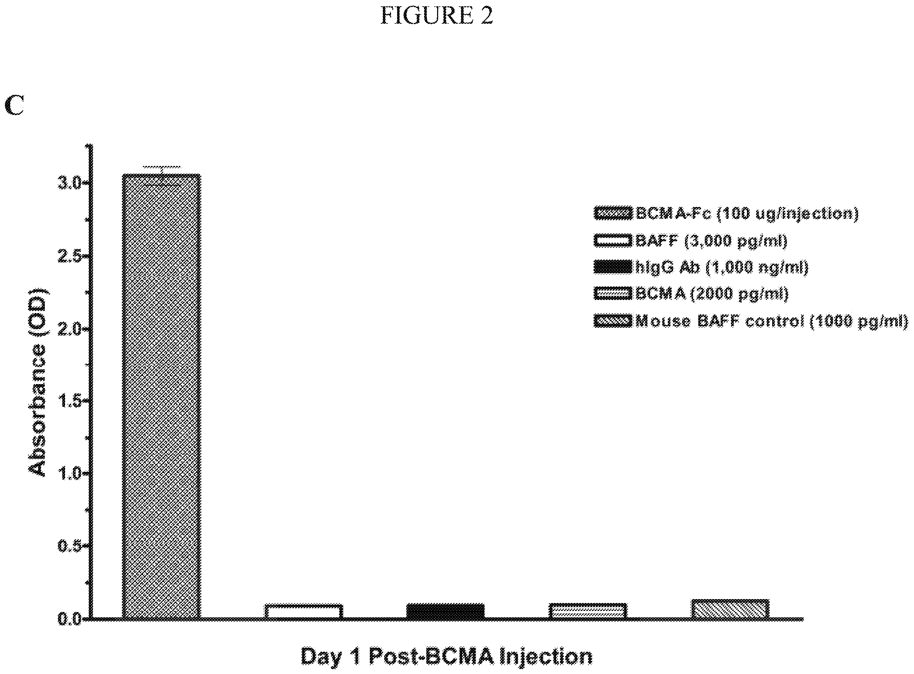

Figure 2 shows that BCMA and BAFF form complexes *in vitro.* **A**, rhBCMA-Fc injected into C57 Bl/6 mice binds mouse BAFF, forming human BCMA (hBCMA)-mBAFF complexes. **B,** hBCMA-mBAFF complexes were detected in severe combined immune deficient (SCID) mice dosed with hBCMA-Fc (top panel) and BAFF levels in these mice following formation of hBCMA-mBAFF complexes (bottom panel). C, hBCMA-mBAFF complexes were detected as early as day 1 in C57 Bl/6 mice dosed with rhBCMA-Fc. Negative controls include samples of mBAFF, hIgG and rhBCMA. Absorbance was determined by a µOuant microplate spectrophotometer with KC Junior software. Data are presented as means ± SEM, obtained from two independent experiments and samples were analyzed twice.

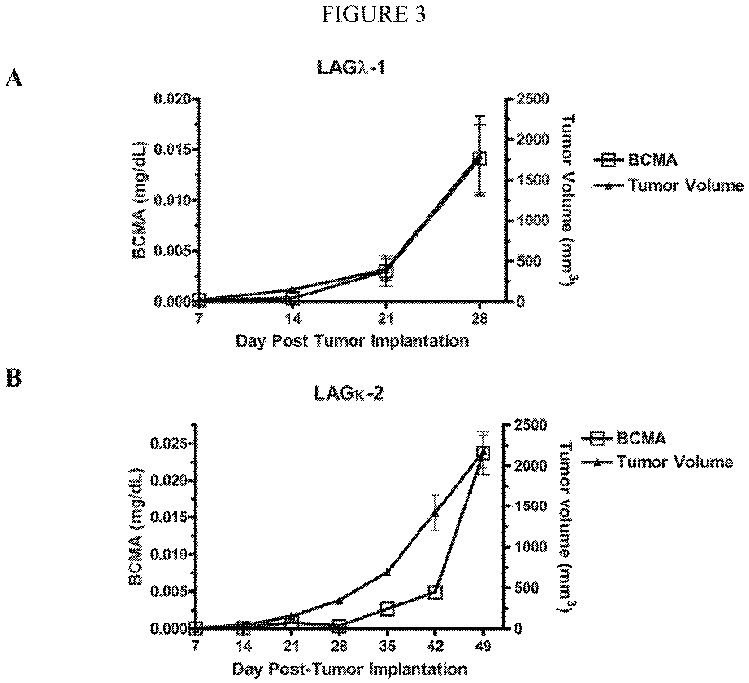

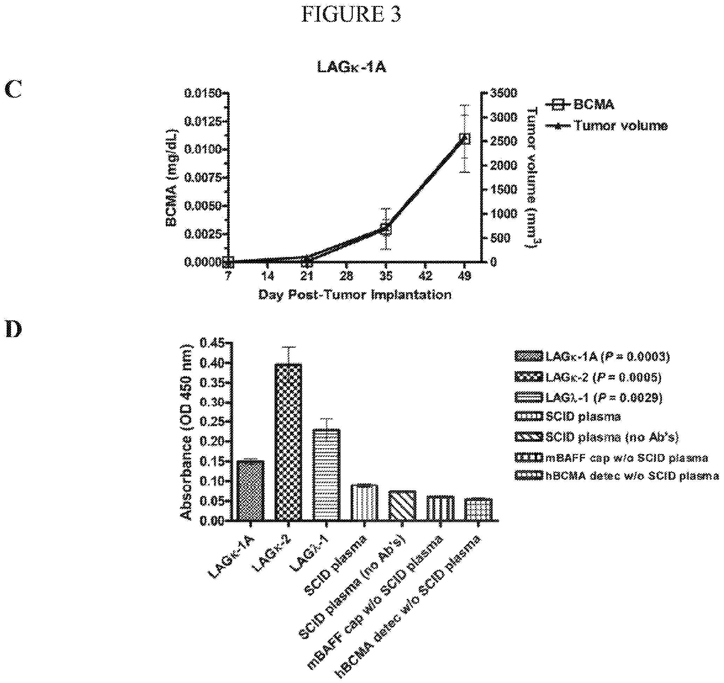

Figure 3 shows that plasma human BCMA levels correlate with human MM tumor volume and BAFF-BCMA complexes are found in SCID mice bearing these tumors: In SCID mice bearing the **A**, EAOPλ-1, **B,** LAGκ-2 and C, LAG κ-1A human MM xenografts. **D,** Human BCMA (from human MM tumors growing in SCID mice) binds mouse BAFF in mouse plasma and forms hBCMA-mBAFF complexes. **D,** In the plasma from mice bearing these three human MM xenografts, the levels of BCMA in the plasma correlate with the amount of BAFF-BCMA complex formation.

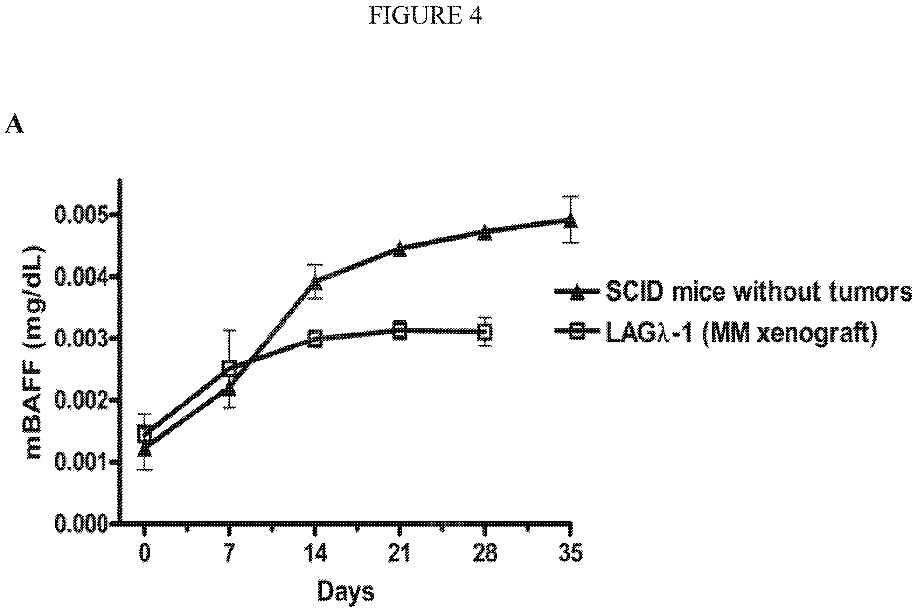

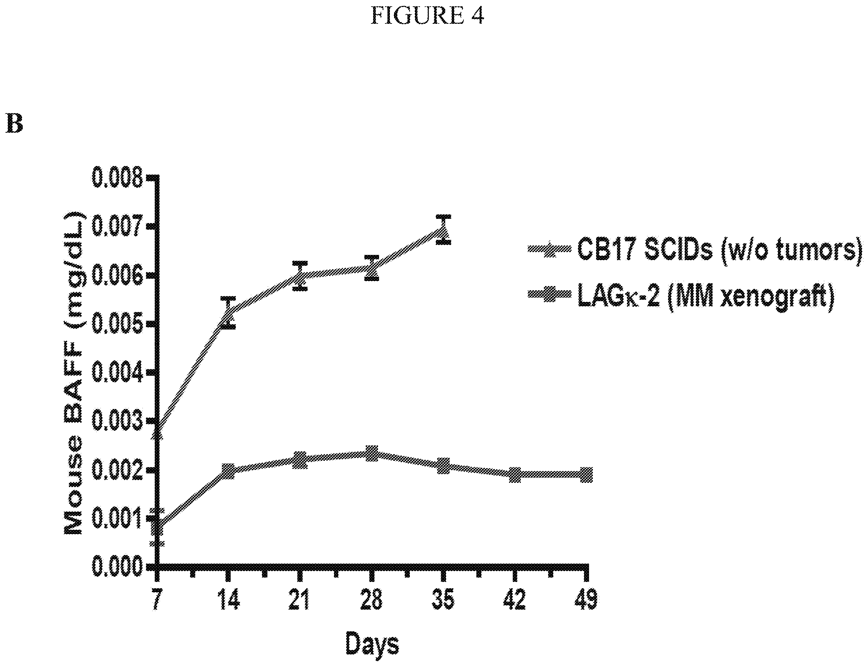

Figure 4 shows that severe combined immunodeficiency (SCID) mice bearing human multiple myeloma (MM) xenografts show reduced plasma BAFF levels. **A**, Decrease in plasma BAFF levels in mice implanted with the human MM tumor LAGλ-1 when compared to SCID mice without MM tumors (day 14: *P* = 0.0143; day 21: *P* = 0.0002; and day 28: *P* = 0.0008). **B,** Decrease in plasma BAFF levels in mice implanted with the human MM tumor LAGκ-2 when compared to SCID mice without MM tumor (day 14: *P* = 0.0015; day 21: *P* < 0.0001; day 28: *P* < 0.0001; and day 35: *P* < 0.0001). Results presented are group means ± SEM (n = 4-5 mice/group) from two independent experiments.

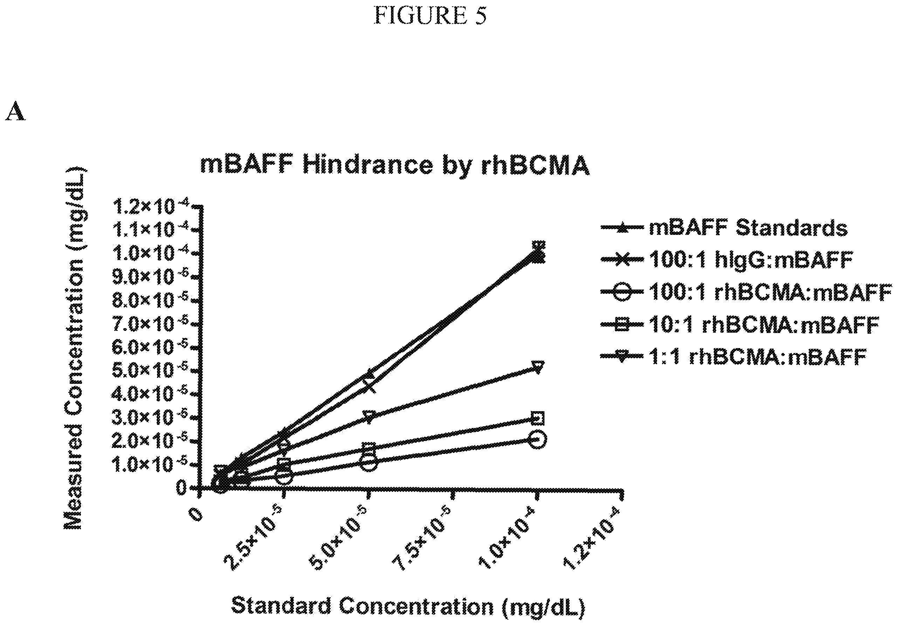

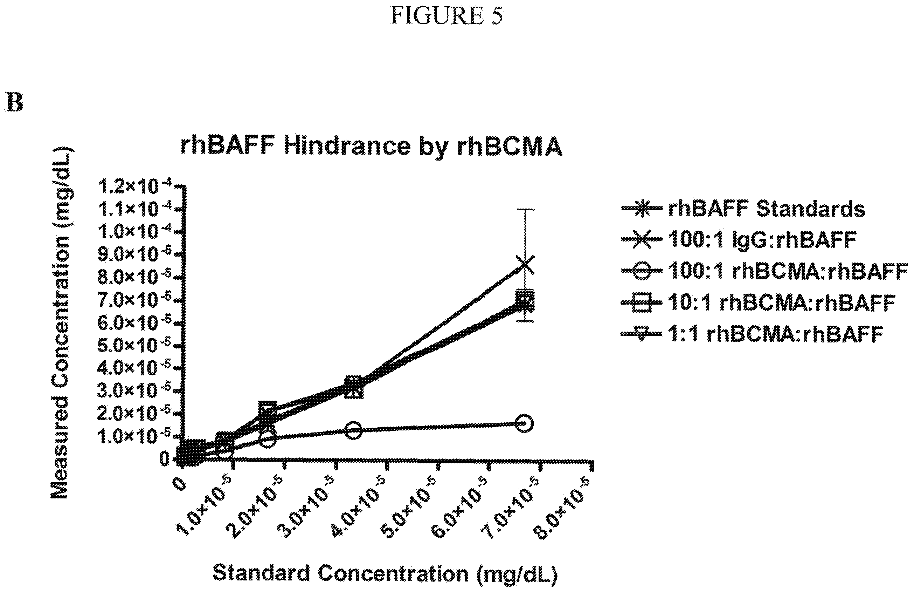

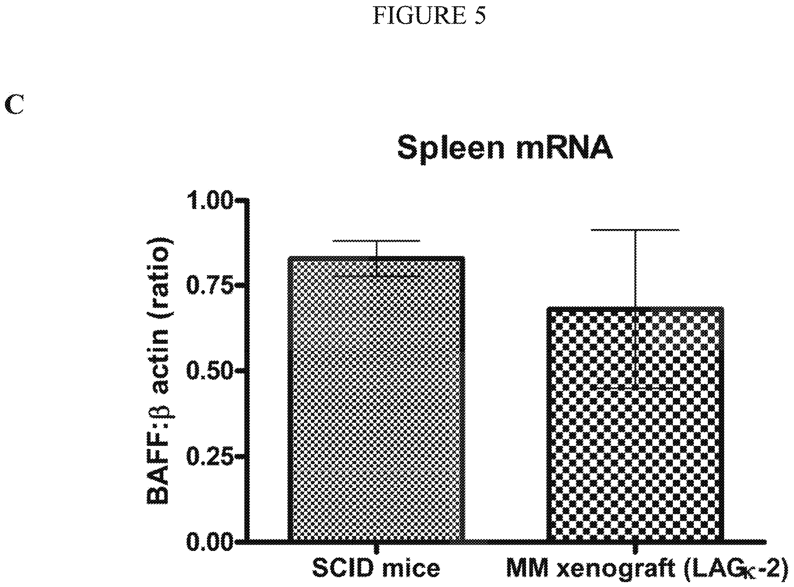

Figure 5 shows that increasing concentrations of rhBCMA interfere with mouse BAFF detection (**A**) and human BAFF detection (**B**), with the highest concentration of BCMA used being similar to concentrations found in MM patients. **C**, Using RT-PCR, no differences in BAFF mRNA levels were detected in SCID mice with or without human MM xenografts.

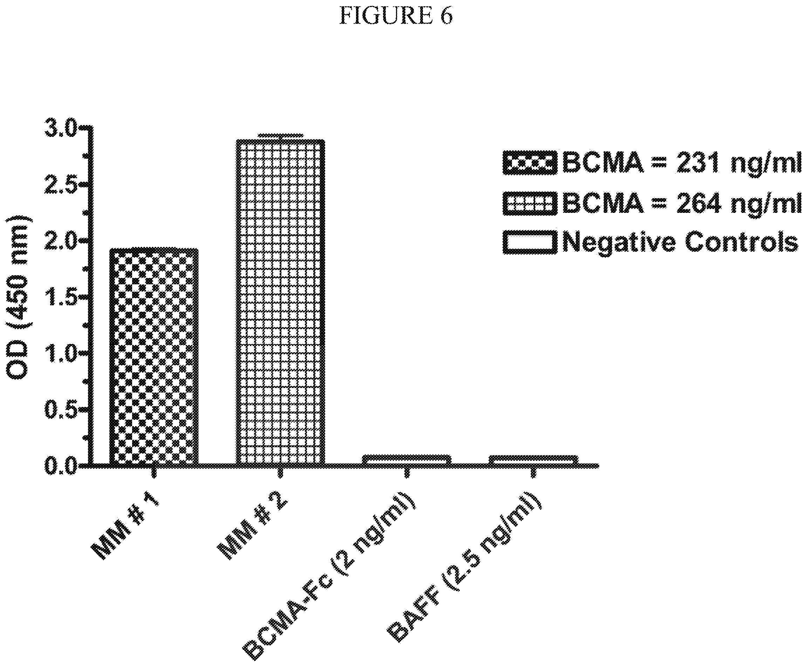

Figure 6 shows that sera of MM patients contain hBCMA-hBAFF complexes. BCMA-BAFF complexes in serum of MM patients were detected using anti-human BAFF capture antibody and anti-hBCMA detection antibody.

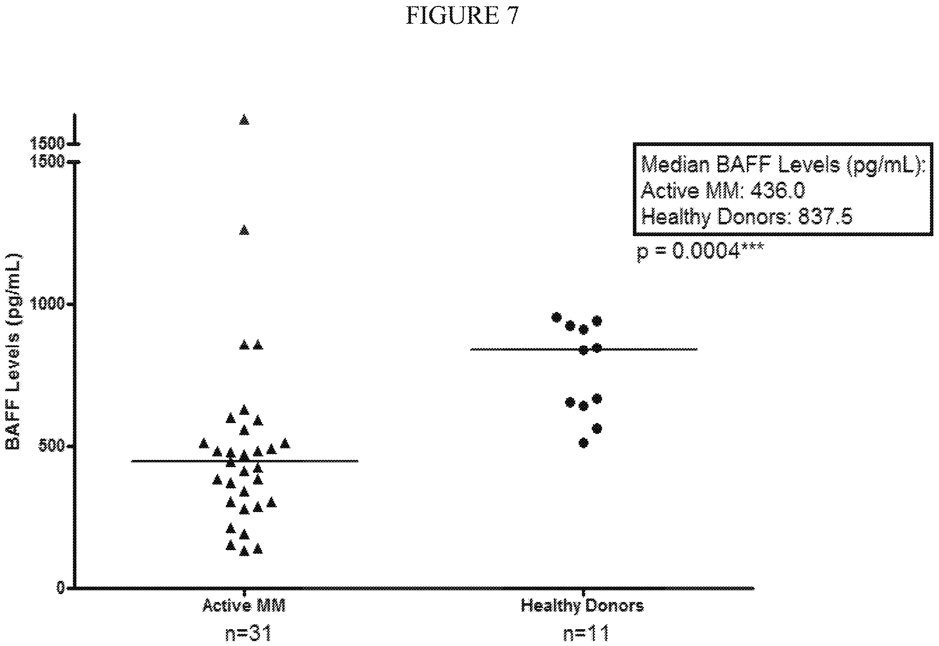

Figure 7 shows that serum BAFF levels are reduced in MM patients with active disease compared to healthy donors. Determination of serum BAFF levels from 31 patients with active MM (13 with progressive disease (PD) and 18 untreated) versus healthy donors (n = 11) shows reduced BAFF levels in the MM patients (436.0 pg/mL) compared to healthy donors (837.5 pg/mL).

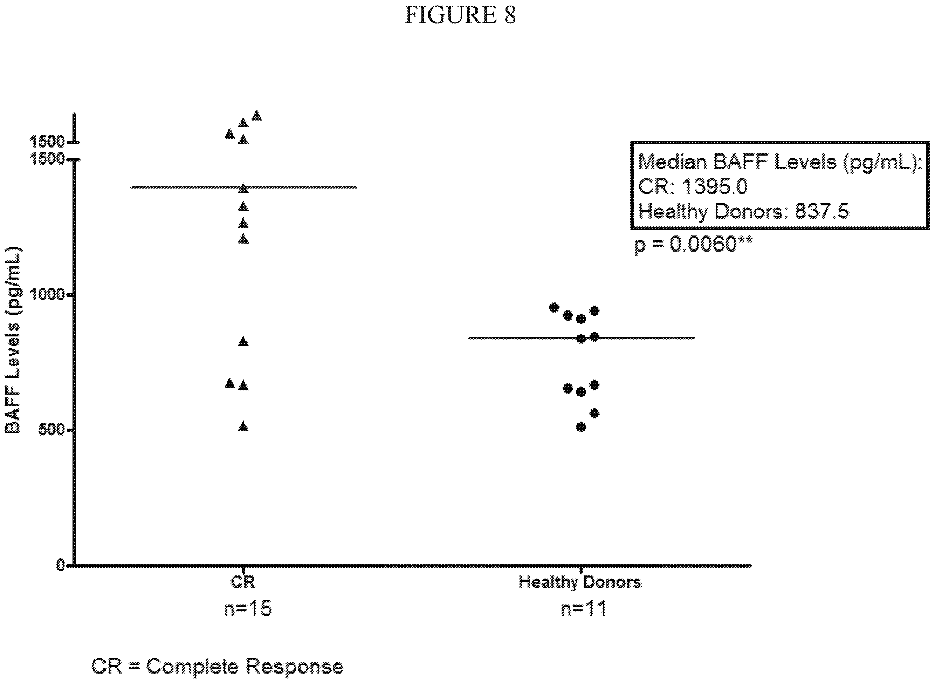

Figure 8 shows serum BAFF levels are increased in MM patients in complete remission (CR) compared to healthy donors. Determination of serum BAFF levels from 15 patients with active MM versus healthy donors (n = 11) shows increased BAFF levels in the MM patients (1395.0 pg/mL) compared to healthy donors (837.5 pg/mL).

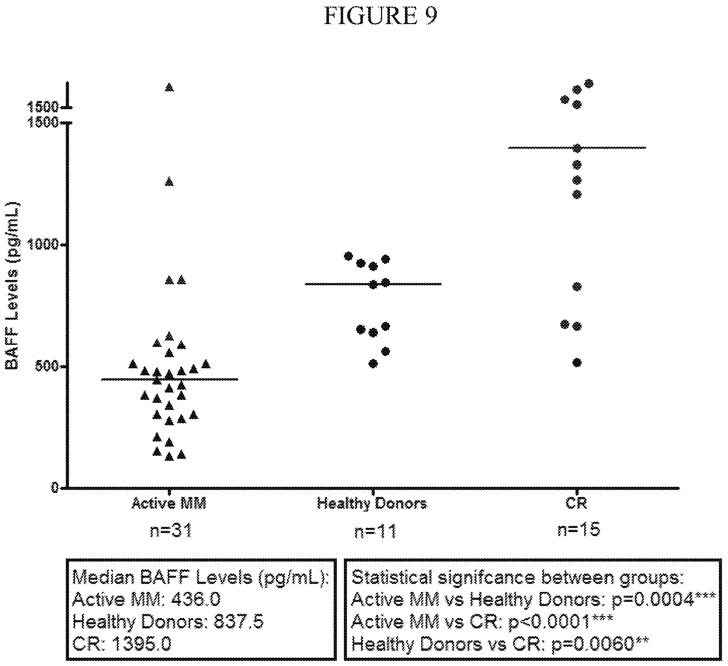

Figure 9 shows that serum BAFF levels can be used to monitor multiple myeloma. Serum BAFF levels are reduced in MM patients with active disease compared to healthy donors, and increased in MM patients in complete remission (CR) compared to healthy donors.

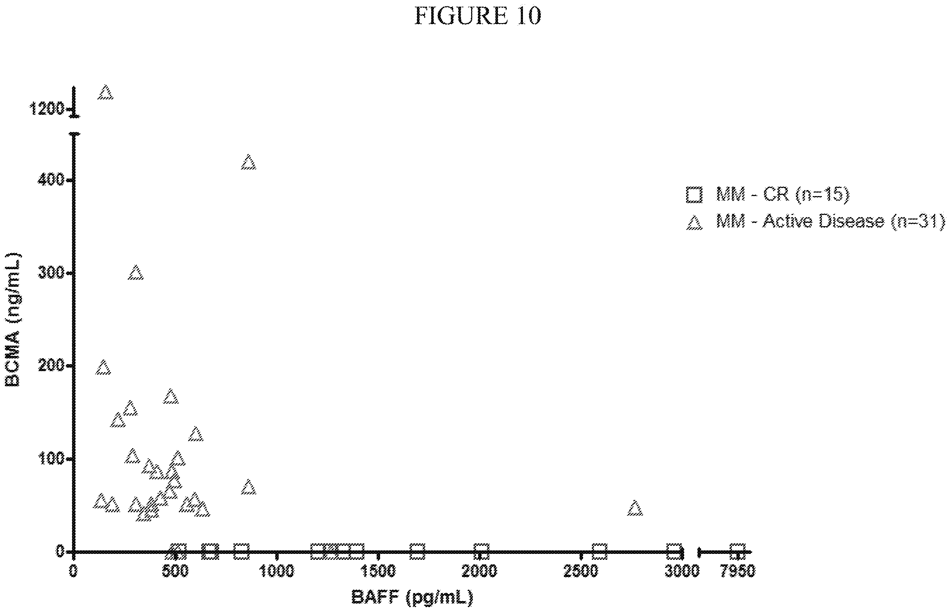

Figure 10 shows that serum B cell maturation antigen (BCMA) levels are inversely related to serum BAFF levels in MM patients.

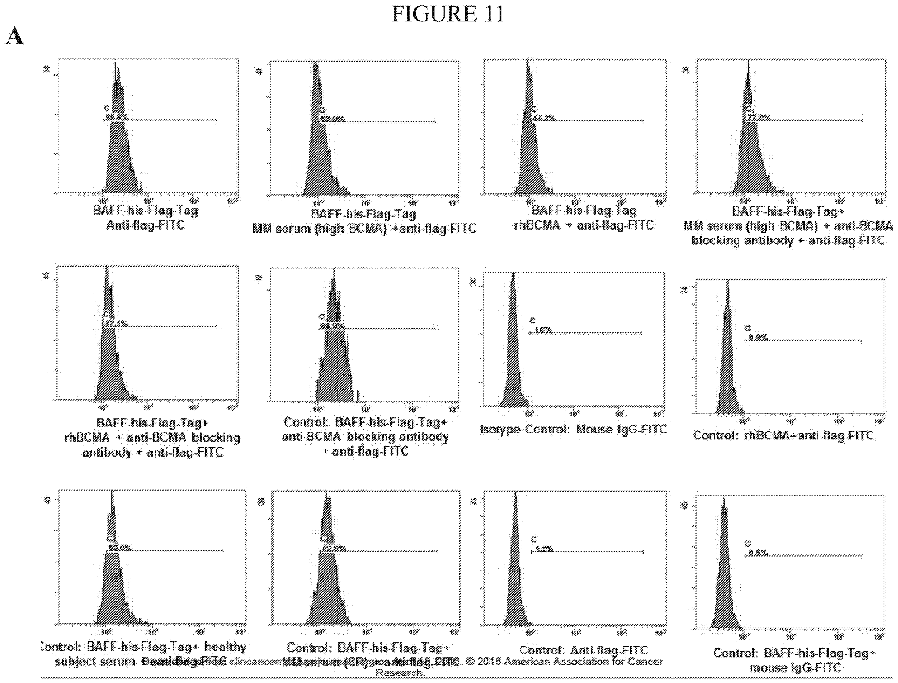

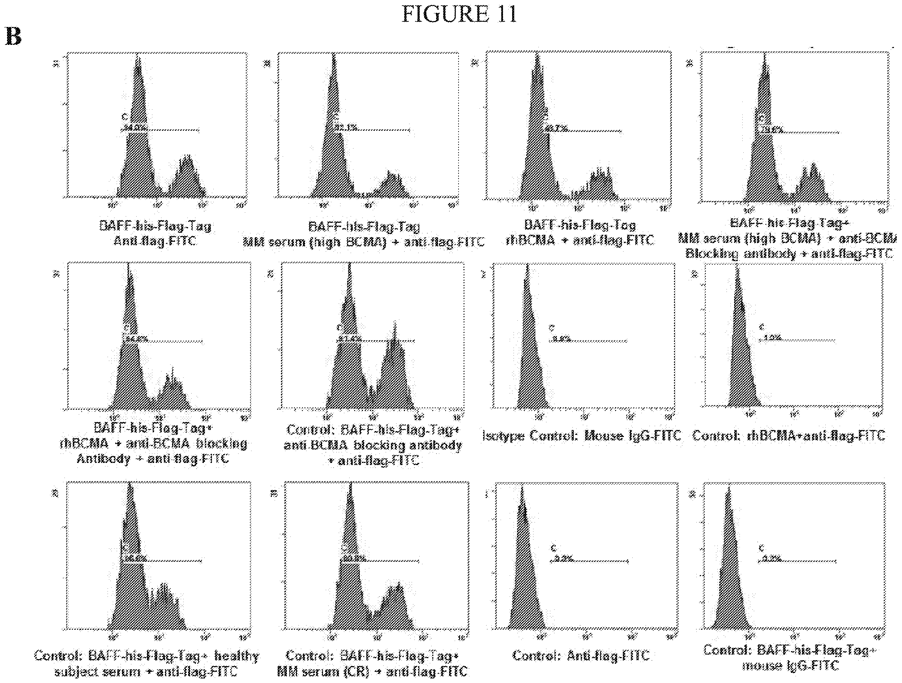

Figure 11 shows flow cytometric analysis of serum BAFF (sBAFF; 500 ng/mL) binding to Raji cells (**A**) in the presence of rhBCMA (3.0 µg/mL, **A**, top row, third column) and serum from MM patients with progressive disease (PD) with a high level of serum BCMA (0.75 µg/mL, **A**, top row, second column) or complete remission (CR) with a low level (0.02 µg/mL, **A**, bottom row, second column) or a healthy subject (also 0.02 µg/mL, **A**, bottom row, first column). Both rhBCMA and serum from the MM patient with PD with a high level of serum BCMA markedly reduced BAFF binding to Raji B-cells, and addition of anti-BCMA blocking antibody restored BAFF binding. In contrast, minimal reduction in BAFF binding occurred in the presence of serum from the healthy subject (**A**, bottom row, first column) or MM patient in CR (**A**, bottom row, second column) both with low serum BCMA levels. **B,** This same experiment was duplicated using an additional human B-cell line, TB94, and similar results were obtained. Flow cytometric analysis was performed using a Beckman-Coulter FC500 cytometer with Cytomics CXP software. Each data point represents one individual MM patient's serum sample.

### DETAILED DESCRIPTION

Currently, an objective test to determine the immune status of a subject, specifically monitoring MM, is not available, and the existence of such a disease is determined by a physician's observation of the subject's physical symptoms (e.g., body temperature or physical discomfort such as pain). However, such observations are subjective and can vary from one physician to another. Furthermore, a rapid and reliable determination of a subject's response to treatment is also currently not available, and would be greatly facilitated by a test that could reliably monitor a subject's immune status at different time points during the course of a treatment regimen. The present inventors have found that levels of serum or plasma BAFF polypeptide correlate with multiple myeloma in a subject. The present inventors specifically have found that an increased amount of serum or plasma BAFF polypeptide in a serum or plasma sample obtained from a subject compared to BAFF polypeptide in a control sample obtained from a normal healthy subject is indicative of a multiple myeloma (MM) patient who has achieved remission of MM, wherein the BAFF polypeptide comprises an amino acid sequence having at least 75% identity with SEQ ID NO: 1. Further disclosed herein for illustration purposes only is that while a decreased amount of BAFF polypeptide or a fragment thereof in a biological sample obtained from a subject compared to amounts of BAFF polypeptide or a fragment thereof in a biological sample obtained from a normal healthy subject indicates that the subject is suffering or is at higher risk to suffer from an infection or an immune deficiency-related disease.

In various aspects of said disclosure present for illustration purposes only, methods for reliably monitoring the immune status of a subject are provided. Concentrations of serum or plasma BAFF polypeptide as defined herein in a serum or plasma sample is detected and/or measured and compared in the use of the invention against a baseline or control to reliably monitor multiple myeloma of a subject. Without wishing to be bound to a particular theory, it is believed that because lower levels of BAFF polypeptide or a fragment thereof were detected in biological samples of subjects having active disease compared to biological samples of subjects having indolent disease, whereas higher levels of BAFF polypeptide or a fragment thereof were detected in biological samples of subjects having impaired immune systems compared to biological samples of normal healthy subjects, levels of soluble serum or plasma BAFF polypeptide can be used in the invention to reliably monitor multiple myeloma of a subject.

In various other aspects of the disclosure for illustration purposes only, methods for reliably monitoring the response of a subject to treatments targeted to improve the immune status of the subject are provided. Levels of soluble BAFF polypeptide or a fragment thereof are used to monitor the response of a subject to treatments targeted to improve the immune status of a subject. Without wishing to be bound to a particular theory, it is believed that because levels of BAFF polypeptide or a fragment thereof in a biological sample obtained from a subject correlated with the immune status of the subject, the levels of BAFF polypeptide or a fragment thereof in the biological sample can be determined at different times point subsequent to start of the treatment and compared to an initial time point prior to start of the treatment to monitor the response of a subject to treatments targeted to improve the immune status of the subject.

The practice of the invention will employ, unless indicated specifically to the contrary, conventional methods of chemistry, biochemistry, organic chemistry, molecular biology, microbiology, recombinant DNA techniques, genetics, immunology, and cell biology that are within the skill of the art, many of which are described below for the purpose of illustration. Such techniques are explained fully in the literature. See, *e.g*., Sambrook, et al., Molecular Cloning: A Laboratory Manual (3rd Edition, 2001); Sambrook, et al., Molecular Cloning: A Laboratory Manual (2nd Edition, 1989); Maniatis et al., Molecular Cloning: A Laboratory Manual (1982); Ausubel et al., Current Protocols in Molecular Biology (John Wiley and Sons, updated July 2008); Short Protocols in Molecular Biology: A Compendium of Methods from Current Protocols in Molecular Biology, Greene Pub. Associates and Wiley-Interscience; Glover, DNA Cloning: A Practical Approach, vol. I & II (IRL Press, Oxford, 1985); Anand, Techniques for the Analysis of Complex Genomes, (Academic Press, New York, 1992); Transcription and Translation (B. Hames & S. Higgins, Eds., 1984); Perbal, A Practical Guide to Molecular Cloning (1984); and Harlow and Lane, Antibodies, (Cold Spring Harbor Laboratory Press, Cold Spring Harbor, N.Y., 1998).

### A. Definitions

Unless defined otherwise, all technical and scientific terms used herein have the same meaning as commonly understood by those of ordinary skill in the art to which the invention belongs. For the purposes of the present invention, the following terms are defined below.

The articles "a," "an," and "the" are used herein to refer to one or to more than one *(i.e.,* to at least one) of the grammatical object of the article. By way of example, "an element" means one element or more than one element.

As used herein, the term "about" or "approximately" refers to a quantity, level, value, number, frequency, percentage, dimension, size, amount, weight or length that varies by as much as 30, 25, 20, 25, 10, 9, 8, 7, 6, 5, 4, 3, 2, or 1 % to a reference quantity, level, value, number, frequency, percentage, dimension, size, amount, weight or length. In particular embodiments, the terms "about" or "approximately" when preceding a numerical value indicates the value plus or minus a range of 15%, 10%, 5%, or 1%.

Throughout this specification, unless the context requires otherwise, the words "comprise", "comprises" and "comprising" will be understood to imply the inclusion of a stated step or element or group of steps or elements but not the exclusion of any other step or element or group of steps or elements. By "consisting of' is meant including, and limited to, whatever follows the phrase "consisting of." Thus, the phrase "consisting of' indicates that the listed elements are required or mandatory, and that no other elements may be present. By "consisting essentially of' is meant including any elements listed after the phrase, and limited to other elements that do not interfere with or contribute to the activity or action specified in the disclosure for the listed elements. Thus, the phrase "consisting essentially of' indicates that the listed elements are required or mandatory, but that no other elements are optional and may or may not be present depending upon whether or not they affect the activity or action of the listed elements.

Reference throughout this specification to "one embodiment," "an embodiment," "embodiment," "a particular embodiment," "a related embodiment," "a certain embodiment," "an additional embodiment," "some embodiments," "certain embodiments," "other embodiments," "additional embodiments," "Further embodiments," or "a further embodiment" or combinations thereof means that a particular feature, structure or characteristic described in connection with the embodiment is included in at least one embodiment of the present invention. Thus, the appearances of the foregoing phrases in various places throughout this specification are not necessarily all referring to the same embodiment. Furthermore, the particular features, structures, or characteristics may be combined in any suitable manner in one or more embodiments.

As used herein, the term "BAFF" or "BAFF polypeptide" is intended to generically refer to both the wild-type and variant B-cell activation factor, unless specifically denoted otherwise. BAFF polypeptides are encoded by the BAFF gene. As it is commonly used in the art, the term "gene" is intended to refer to the genomic region encompassing 5' untranslated region(s) (UTR), exons, introns, and 3' UTR. Individual segments may be specifically referred to, e.g., promoter, coding region, etc. Combinations of such segments that provide for a complete BAFF protein may be referred to generically as a protein coding sequence.

The terms "BAFF" or "BAFF polypeptide" or "soluble BAFF polypetide" are used interchangeably and encompass an amino acid sequence encoded by an open reading frame (ORF) of a known BAFF polynucleotide, including the full-length native polypeptide and fragments thereof, namely biologically active fragments and/or fragments corresponding to functional domains, e.g., a region or domain having biological activity, *etc*.; antigenic fragments thereof, and including fusions of the subject polypeptides to other proteins or parts thereof. The amino acid sequence of BAFF polypeptide has been disclosed. (*See e.g*., Schneider et al., J. Exp. Med. 189(11)2: 1747-17564, 1999; Karpusas et al., J. Mol. Biol., 315(5): 1145-1154 (2002). The BAFF polypeptides of the invention is isolated from serum or plasma.

As used herein, the term "fragment thereof' as used in the present disclosure refers to a portion of the full-length native BAFF polypeptide. In some embodiments, the BAFF fragment is a cleaved BAFF polypeptide. In some embodiments, the cleaved BAFF polypeptide is a soluble form of the BAFF polypeptide.

In some embodiments of the invention, the serum or plasma BAFF polypeptide comprises an amino acid sequence having at least 75% identity, at least about 80% identity, at least about 90% identity, at least about 95% identity, at least about 96% identity, at least about 97% identity, at least about 98% identity, or at least about 99% identity with the full-length native BAFF polypeptide.

As used herein, the term "immune status" of a subject refers to the efficiency of the subject's immune system. As such, the immune status of a subject indicates whether the subject's immune system is normal, impaired (for *e.g*., if the subject is afflicted with an immune deficiency disease), or hyperactive (for *e.g*., if the subject is afflicted with a disease, an autoimmune disease, or an illness) compared to a normal healthy subject.

The term "immune system" refers to a system of many biological structures and processes within an organism that protects against disease.

The following are non-limiting embodiments of polynucleotides: a gene or gene fragment, exons, introns, mRNA, tRNA, rRNA, ribozymes, cDNA, recombinant polynucleotides, branched polynucleotides, plasmids, vectors, isolated DNA of any sequence, isolated RNA of any sequence, nucleic acid probes, and primers. A nucleic acid molecule may also comprise modified nucleic acid molecules, such as methylated nucleic acid molecules and nucleic acid molecule analogs. Analogs of purines and pyrimidines are known in the art. Nucleic acids may be naturally occurring, *e*.*g*., DNA or RNA, or may be synthetic analogs, as known in the art. Such analogs may be preferred for use as probes because of superior stability under assay conditions. Modifications in the native structure, including alterations in the backbone, sugars or heterocyclic bases, have been shown to increase intracellular stability and binding affinity. Among useful changes in the backbone chemistry are phosphorothioates; phosphorodithioates, where both of the non-bridging oxygens are substituted with sulfur; phosphoroamidites; alkyl phosphotriesters and boranophosphates. Achiral phosphate derivatives include 3'-O'-5'-S-phosphorothioate, 3'-S-5'-O-phosphorothioate, 3'-CH2-5'-O-phosphonate and 3'-NH-5'-O-phosphoroamidate. Peptide nucleic acids replace the entire ribose phosphodiester backbone with a peptide linkage.

The terms "polypeptide" and "protein", used interchangeably herein, refer to a polymeric form of amino acids of any length, which can include coded and non-coded amino acids, chemically or biochemically modified or derivatized amino acids, and polypeptides having modified peptide backbones. In various embodiments, BAFF polypeptides are contemplated for use within diagnostic, prognostic, or monitoring compositions and methods disclosed herein. The term includes fusion proteins, including, but not limited to, fusion proteins with a heterologous amino acid sequence, fusions with heterologous and homologous leader sequences, with or without N-terminal methionine residues; immunologically tagged proteins; and the like.

A "substantially isolated" or "isolated" substance is one that is substantially free of its associated surrounding materials in nature. By substantially free is meant at least 50%, preferably at least 70%, more preferably at least 80%, and even more preferably at least 90% free of the materials with which it is associated in nature. As used herein, an "isolated" can refer to polynucleotides, polypeptides, cells, samples, and antibodies.

Hybridization reactions can be performed under conditions of different "stringency". Conditions that increase stringency of a hybridization reaction are widely known and published in the art. *See*, for example, Sambrook *et al.* (1989). Examples of relevant conditions include (in order of increasing stringency): incubation temperatures of 25° C., 37° C., 50° C. and 68° C.; buffer concentrations of 10×SSC, 6×SSC, 1×SSC, 0.1×SSC (where SSC is 0.15 M NaCl and 15 mM citrate buffer) and their equivalents using other buffer systems; formamide concentrations of 0%, 25%, 50%, and 75%; incubation times from 5 minutes to 24 hours; 1, 2, or more washing steps; wash incubation times of 1, 2, or 15 minutes; and wash solutions of 6×SSC, 1×SSC, 0.1×SSC, or deionized water. Examples of stringent conditions are hybridization and washing at 50° C. or higher and in 0.1×SSC (9 mM NaCl/0.9 mM sodium citrate).

The term "target cell" includes an individual cell, cell from a biological sample, or cell culture. Target cells include progeny of a single target cell, and the progeny may not necessarily be completely identical (in morphology or in total DNA complement) to the original parent cell due to natural, accidental, or deliberate mutation and/or change. In particular embodiments, target cells include multiple myeloma, chronic lymphocytic leukemia, lymphoma, or Waldenstrom's macroglobulinemia tumor cells, bone marrow or peripheral blood mononuclear cells, B-cells, or plasma cells.

The detection systems as used in the in vitro use of the invention are based, in part, on the ability of a binding agent to bind soluble serum or plasma BAFF polypeptide as defined herein. Generally, the invention contemplates the use of a binding agent that specifically binds soluble serum or plasma BAFF polypeptide as defined herein, resulting in the formation of a detectable complex of soluble BAFF polypeptide and binding agent. In some embodiments, the invention utilizes two binding agents, a capture binding agent and a detection binding agent, both of which bind to soluble serum or plasma BAFF polypeptide as defined herein, resulting in the formation of a ternary complex comprising capture binding agent, soluble BAFF polypeptide, and detection binding agent.

Any of a variety of binding agents may be used, including, for example, polypeptides, sugars, and nucleic acids. In yet other embodiments, the invention further includes the use of an additional binding agent that binds to the detection binding agent. Such an additional binding agent may be useful, e.g., in detecting bound detection binding agent. Accordingly, one example of such an additional binding agent is antibodies specific for a fragment of an antibody, e.g., an F_{c} fragment, which may be detectably labeled and, therefore used to detect bound detection binding agent, and are particularly useful when the detection binding agent is not itself easily amenable to labeling. In certain embodiments, the binding agent is an antibody specific for bacteria.

The term "binds specifically," in the context of antibody binding, refers to high avidity and/or high affinity binding of an antibody to a specific polypeptide *i.e*., epitope of a BAFF polypeptide or a fragment thereof. Antibody binding to an epitope on a specific polypeptide (also referred to herein as "an epitope") is preferably stronger than binding of the same antibody to any other epitope, particularly those which may be present in molecules in association with, or in the same sample, as the specific polypeptide of interest, e.g., binds more strongly to a specific epitope on the soluble BAFF polypeptide than to a different BAFF epitope or non-BAFF epitope. Antibodies which bind specifically to a polypeptide of interest may be capable of binding other polypeptides at a weak, yet detectable, level (e.g., 10% or less, 5% or less, 1% or less of the binding shown to the polypeptide of interest). Such weak binding, or background binding, is readily discernible from the specific antibody binding to the compound or polypeptide of interest, e.g. by use of appropriate controls. In general, antibodies used in the in vitro use of the invention which bind to a specific serum or plasma BAFF polypeptide as defined herein with a binding affinity of 10⁷ moles/L or more, preferably 10⁸ moles/L or more are said to bind specifically to the specific BAFF polypeptide. In general, an antibody with a binding affinity of 10⁶ moles/L or less is not useful in that it will not bind an antigen at a detectable level using conventional methodology currently used.

In some embodiments, the affinity of specific binding of a BAFF binding agent to soluble BAFF polypeptide or a fragment thereof is about 2 times greater than background binding, about 5 times greater than background binding, about 10 times greater than background binding, about 20 times greater than background binding, about 50 times greater than background binding, about 100 times greater than background binding, or about 1000 times greater than background binding or more.

In other embodiments, the affinity of specific binding is between about 2 to about 1,000 times greater than background binding, between about 2 to 500 times greater than background binding, between about 2 to about 100 times greater than background binding, between about 2 to about 50 times greater than background binding, between about 2 to about 20 times greater than background binding, between about 2 to about 10 times greater than background binding, between about 5 to about 100 times greater than background binding, between about 5 to about 50 times greater than background binding, between about 5 to about 20 times greater than background binding, between about 10 to about 100 times greater than background binding, between about 10 to about 50 times greater than background binding, between about 50 to about 500 times greater than background binding, or any intervening range of affinity.

Accordingly, specific binding occurs between a binding agent and soluble BAFF polypeptide or a fragment thereof where there is an interaction between the two which produces a bound complex having the characteristics of an antibody/antigen or enzyme/substrate interaction. In some embodiments, specific binding is characterized when one member of a pair substantially binds to a particular species and to no other species within the family of compounds to which the corresponding member of the binding member belongs. In other embodiments, specific binding is characterized when one member of a pair substantially binds to one or more particular species and to no other species within the family of compounds to which the corresponding member of the binding member belongs. In yet other embodiments, specific binding is characterized when one member of a pair substantially binds to 1, 2, 3, 4, 5, 6, 7, 8, 9, 10, or more particular species and to no other species within the family of compounds to which the corresponding member of the binding member belongs.

Generally speaking, the binding affinity of a binding agent of the invention (A) to soluble serum or plasma BAFF polypeptide as defined herein(B) can be generally expressed by the chemical equilibrium constant K_{d} resulting from the following reaction: [A] +[B]- [AB]. The chemical equilibrium constant K_{d} is then given by: K_{d}=[A]x[B]/[AB]. Whether the binding of a binding agent is specific or not can be judged from the difference between the binding affinity (K_{d} value) of the binding agent to soluble BAFF polypeptide or a fragment thereof, versus the binding to another polypeptide.

K_{d} values and differences in K_{d} values can be measured using, for example, *in vitro* or *in vivo* binding assays and/or assays on other materials such as a polystyrene microtitre plate or a specialized surface in an analytical biosensor. In some embodiments, the difference between the K_{d} value of a binding agent to soluble BAFF polypeptide or a fragment thereof, versus the binding to an undesired polypeptide is about 2-fold, about 3-fold, about 4-fold, about 5-fold, about 6-fold, about 7-fold, about 8-fold, about-9 fold, about 10-fold, about 20-fold, about 50-fold, about 100-fold, about 1000-fold, or more.

In other embodiments, the K_{d} value is less than 10⁴ M, less than 10⁵ M, less than 10⁶ M, less than 10⁷ M, less than 10⁸ M, less than 10⁹ M, less than 10¹⁰ M and could be 10¹¹ M, less than 10¹² M, less than 10¹³ M, less than 10¹⁴ M, less than 10¹⁵ M or less.

In other aspects, the K_{d} value is between about 10⁴ M and about 10¹⁵ M, between about 10⁴ M and about 10¹² M, between about 10⁴ M and about 10¹⁰ M, between about 10⁶ M and about 10¹⁵ M, between about 10⁶ M and about 10¹² M, between about 10⁶ M and about 10¹⁰ M, between about 10⁸ M and about 10¹⁵ M, between about 10⁸ M and about 10¹² M, between about 10⁸ M and about 10¹⁰ M, between about 10⁷ M and about 10¹⁰ M, or any intervening range of affinity.

The term "antibody" herein is used in the broadest sense and specifically covers monoclonal antibodies, polyclonal antibodies, multispecific antibodies (e.g., bispecific antibodies) formed from at least two intact antibodies, and antibody fragments so long as they exhibit the desired biological activity.

The term "monoclonal antibody" as used herein refers to an antibody obtained from a population of substantially homogeneous antibodies, *i.e*., the individual antibodies comprising the population are identical except for possible naturally occurring mutations that can be present in minor amounts.

In some embodiments, the monoclonal antibody is an anti-BAFF monoclonal antibody. In other embodiments, the monoclonal antibody specifically recognizes an epitope present in a fragment of the soluble BAFF polypeptide.

Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature, 256: 495 (1975), or may be made by recombinant DNA methods (see, e.g., U.S. Pat. No. 4,816,567). The "monoclonal antibodies" may also be isolated from phage antibody libraries using the techniques described in Clackson et al., Nature, 352: 624-628 (1991) and Marks et al., J. Mol. Biol., 222: 581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain (s) is identical with or homologous to corresponding sequences in antibodies derived from another species or belonging to another antibody class or subclass, as well as fragments of such antibodies, so long as they exhibit the desired biological activity (U.S. Pat. No. 4,816,567; Morrison et al., Proc. Natl. Acad. Sci. USA, 81: 6851-6855 (1984)). Methods of making chimeric antibodies are known in the art.

"Humanized" forms of non-human (*e*.*g*., murine) antibodies are chimeric immunoglobulins, immunoglobulin chains or fragments thereof (such as Fv, Fab, Fab', F (ab') 2 or other antigen-binding subsequences of antibodies) which contain minimal sequence derived from non-human immunoglobulin.

For the most part, humanized antibodies are human immunoglobulins (recipient antibody) in which residues from a complementarity-determining region (CDR) of the recipient are replaced by residues from a CDR of a non-human species (donor antibody) such as mouse, rat or rabbit having the desired specificity, affinity, and capacity. In some instances, Fv framework region (FR) residues of the human immunoglobulin are replaced by corresponding non-human residues. Furthermore, humanized antibodies may comprise residues which are found neither in the recipient antibody nor in the imported CDR or framework sequences. These modifications are made to further refine and maximize antibody performance. In general, the humanized antibody will comprise substantially all of at least one, and typically two, variable domains, in which all or substantially all of the hypervariable loops correspond to those of a non-human immunoglobulin and all or substantially all of the FR regions are those of a human immunoglobulin sequence although the FR regions may include one or more amino acid substitutions that improve binding affinity. The number of these amino acid substitutions in the FR is typically no more than 6 in the H chain, and no more than 3 in the L chain. The humanized antibody optimally also will comprise at least a portion of an immunoglobulin constant region (Fc), typically that of a human immunoglobulin. For further details, see Jones et al., Nature, 321: 522-525 (1986); Reichmann et al., Nature, 332: 323-329 (1988); and Presta, Curr. Op. Struct. Biol., 2: 593-596 (1992). The humanized antibody includes a PRIMATIZED antibody wherein the antigen-binding region of the antibody is derived from an antibody produced by, *e*.*g*., immunizing macaque monkeys with the antigen of interest. Methods of making humanized antibodies are known in the art.

Human antibodies can also be produced using various techniques known in the art, including phage-display libraries. Hoogenboom and Winter, J. Mol. Biol., 227: 381 (1991); Marks et al., J. Mol. Biol., 222: 581 (1991). The techniques of Cole *et al.* and Boerner *et al.* are also available for the preparation of human monoclonal antibodies. Cole et al., Monoclonal Antibodies and Cancer Therapy, Alan R. Liss, p. 77 (1985); Boerner et al., J. Immunol., 147(1): 86-95 (1991).

"Functional fragments" of the binding antibodies of the disclosure present as illustration purposes only are those fragments that retain binding to antigen with substantially the same affinity as the intact full chain molecule from which they are derived.

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than about 95% by weight of antibody as determined by the Lowry method, and most preferably more than about 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody in situ within recombinant cells since at least one component of the antibody's natural environment will not be present. Ordinarily, however, isolated antibody will be prepared by at least one purification step.

The terms "detectably labeled antibody" refers to an antibody (or antibody fragment which retains binding specificity for soluble BAFF polypeptide or a fragment thereof), having an attached detectable label. The detectable label is normally attached by-chemical conjugation, but where the label is a polypeptide, it could alternatively be attached by genetic engineering techniques. Methods for production of detectably labeled proteins are well known in the art. Detectable labels may be selected from a variety of such labels known in the art, including, but not limited to, haptens, radioisotopes, fluorophores, paramagnetic labels, enzymes (*e*.*g*., horseradish peroxidase), or other moieties or compounds which either emit a detectable signal (*e*.*g*., radioactivity, fluorescence, color) or emit a detectable signal after exposure of the label to its substrate. Various detectable label/substrate pairs (*e*.*g*., horseradish peroxidase/diaminobenzidine, avidin/streptavidin, luciferase/luciferin)), methods for labeling antibodies, and methods for using labeled antibodies are well known in the art (*see*, for example, Harlow and Lane, eds. (Antibodies: A Laboratory Manual (1988) Cold Spring Harbor Laboratory Press, Cold Spring Harbor, N.Y.)).

In one technique which is not part of the invention and only present as illustration purposes only, an immunogen comprising the polypeptide is initially injected into any of a wide variety of mammals (*e*.*g*., mice, rats, rabbits, sheep or goats). Polyclonal antibodies specific for the polypeptide may then be purified from such antisera by, for example, affinity chromatography using the polypeptide coupled to a suitable solid support. In some embodiments, the antibody is an anti-BAFF polyclonal antibody. In other embodiments, the antibody is polyclonal antibody that recognizes the soluble BAFF polypeptide. In yet other embodiments of said disclosure being present for illustration purposes only, the antibody is polyclonal antibody that recognizes a fragment of the soluble BAFF polypeptide.

A "biological sample" encompasses a variety of sample types obtained from an individual and can be used in a diagnostic or monitoring assay. The definition encompasses blood and other liquid samples of biological origin, solid tissue samples such as a biopsy specimen or tissue cultures or cells derived there from and the progeny thereof. The definition also includes samples that have been manipulated in any way after their procurement, such as by treatment with reagents, solubilization, or enrichment for certain components, such as polynucleotides. The term "biological sample" encompasses a clinical sample, and also includes, without limitation, cells in culture, cell supernatants, cell lysates, serum, plasma, urine, cerebral spinal fluid, biological fluid, and tissue samples. The sample may be pretreated as necessary by dilution in an appropriate buffer solution or concentrated, if desired. Any of a number of standard aqueous buffer solutions, employing one of a variety of buffers, such as phosphate, Tris, or the like, preferably at physiological pH can be used. Biological samples can be derived from patients using well known techniques such as venipuncture, lumbar puncture, fluid sample such as saliva or urine, or tissue biopsy and the like. In the present invention the biological sample used in the in vitro use is serum or plasma.

As used herein, the terms "correlated with" or "associated with" refer to the levels of soluble BAFF polypeptide or a fragment thereof in a biological sample of a subject that has a statistically significant correlation with a physiologic state, e.g., disease status or extent of the disease, response to treatment, and survival. The strength of the correlation between levels of soluble BAFF polypeptide or a fragment thereof and the presence or absence of a particular physiologic state may be determined by a statistical test of significance. Methods for determining the strength of a correlation between the expression level of a differentially-expressed gene and a particular physiologic state by assigning a statistical score to the correlation are reviewed in Holloway et al. (2002) Nature Genetics Suppl. 32:481-89, Churchill (2002) Nature Genetics Suppl. 32:490-95, Quackenbush (2002) Nature Genetics Suppl. 32: 496-501; Slonim (2002) Nature Genetics Suppl. 32:502-08; and Chuaqui et al. (2002) Nature Genetics Suppl. 32:509-514.

A "conjugate" refers to any molecule, e.g., antibody bound or joined covalently or non-covalently to another molecule, e.g., a hapten, small molecule, or label, including fusion proteins and as well as molecules that contain both amino acid or protein portions and non-protein portions. Conjugates may be synthesized by a variety of techniques known in the art including, for example, solid phase synthesis, solution phase synthesis, organic chemical synthetic techniques or a combination of these techniques. The choice of synthesis will depend upon the particular molecule to be generated.

The terms "individual," "subject," and "patient," used interchangeably herein, refer to a mammal, including, but not limited to, murines, simians, humans, mammalian farm animals, mammalian sport animals, and mammalian pets. In some embodiments, the subject is a human subject.

The term "mammal" refers to any animal classified as a mammal, including humans, domestic and farm animals, and zoo, sports, or pet animals, such as dogs, horses, cats, cows, etc. In some embodiments, the mammal herein is human.

### B. Uses for Monitoring Multiple Myeloma of a Subject

The present inventors have discovered that soluble serum or plasma BAFF polypeptide levels correlate with multiple myeloma in a subject. With regard to the in vitro use of the invention, the serum or plasma BAFF polypeptide levels are increased in serum or plasma samples obtained from subjects who have achieved remission compared to levels in healthy control subjects, wherein the BAFF polypeptide comprises an amino acid sequence having at least 75% identity with SEQ ID NO: 1. Particular aspects of the disclosure present for illustration purposes only also provide methods for the monitoring the immune status of a subject as well as monitoring the response of the subject to treatment, based upon the level of BAFF polypeptide or a fragment thereof in a biological sample obtained from a patient, including, e.g., a patient's bloodstream, serum, bone marrow, or tissue at different time points. A variety of methods of determining soluble BAFF polypeptide levels are known and available in the art. These may involve the use of a BAFF binding agent, such as an antibody that binds with specificity to soluble BAFF polypeptide. As discussed elsewhere herein, there are a variety of assay formats known to those of ordinary skill in the art and suitable for using a binding agent to detect polypeptide markers in a sample. *E.g*., ELISA assays, lateral flow assays, etc.; *see also*, Harlow and Lane, Antibodies: A Laboratory Manual, Cold Spring Harbor Laboratory, 1988.

In general, a condition leading to impairment of the immune system is monitored by the presence of at least about 2-fold, at least about 5-fold, at least about 10-fold, at least about 20-fold, at least about 50-fold, at least about 100-fold, at least about 1000-fold, or higher levels of soluble BAFF polypeptide or a fragment thereof as compared to those in a normal control subject. In general, an infective or disease state is monitored by the presence of at least about 2-fold, at least about 5-fold, at least about 10-fold, at least about 20-fold, at least about 50-fold, at least about 100-fold, at least about 1000-fold, or lower levels of soluble BAFF polypeptide or a fragment thereof as compared to those in a normal control subject.

In some aspects which are just present for illustration purposes only, methods of monitoring immune status of a subject comprise: (a) detecting an amount of BAFF polypeptide or a fragment thereof in a biological sample, e.g., serum, obtained from a subject; and (b) comparing the amount of BAFF polypeptide or fragment thereof detected in (a) to a predetermined cut-off value or to an amount detected in a control serum or plasma sample, wherein an increased amount of BAFF polypeptide or a fragment thereof in the biological sample of the subject as compared to the predetermined cut-off value or amount in the control serum or plasma sample is indicative of an impaired immune system, and a decreased amount of BAFF polypeptide or a fragment thereof in the biological sample of the subject as compared to the predetermined cut-off value or amount in the control serum or plasma sample indicates that the subject is at higher risk of or is suffering from an infection or a disease, wherein the biological sample is a serum or plasma sample.

In other aspects which are just present for illustration purposes only, methods of monitoring immune status of a subject comprise: (a) detecting an amount of BAFF or a fragment thereof in a biological sample, e.g., serum, obtained from a subject; and (b) comparing the amount of BAFF polypeptide or a fragment thereof detected in (a) to a predetermined cut-off value or to an amount detected in a control serum or plasma sample, wherein a decreased amount of BAFF polypeptide or a fragment thereof in the biological sample of the subject as compared to the predetermined cut-off value or amount in the control serum or plasma sample is indicative of an impaired immune system, wherein the biological sample is a serum or plasma sample.

In yet other aspects which are just present for illustration purposes only, methods of monitoring immune status of a subject comprise: (a) detecting an amount of BAFF polypeptide or a fragment thereof in a biological sample obtained from a subject at an initial time point; (b) detecting an amount of BAFF polypeptide or a fragment thereof in the biological sample obtained from the subject at a later time point; and (c) comparing the amount of BAFF polypeptide or a fragment thereof detected in (a) to the amount of BAFF polypeptide or a fragment thereof detected in (b), wherein an increased amount of BAFF polypeptide or a fragment thereof detected in (b) as compared to the amount of BAFF polypeptide or a fragment thereof detected in (a) indicates that the subject is responding to treatment, and wherein a decreased or unchanged amount of BAFF polypeptide or a fragment thereof detected in (b) as compared to the amount of BAFF polypeptide or a fragment thereof detected in (a) indicates that the subject is not responding to treatment, wherein the biological sample is a serum or plasma sample.

In some aspects which are just present for illustration purposes only, the immune status of a subject may be determined by (a) contacting a biological sample obtained from a subject with a BAFF binding agent; (b) detecting in the sample a level of soluble BAFF polypeptide or a fragment thereof that binds to the binding agent; and (c) comparing the level of soluble BAFF polypeptide or a fragment thereof with a predetermined cut-off value or with the value obtained from a normal control subject. In certain aspects, the cut-off value for the detection of impairment of the immune system and/or an infection or disease state is the average mean signal obtained when the immobilized antibody is incubated with samples from patients not suffering from an immune system impairment and not suffering from an infection or disease state. In the present invention, the biological sample of the in vitro use is serum or plasma.

In the invention, detection involves determining soluble serum or plasma BAFF polypeptide levels present in the sample. In certain embodiments, detection is performed using an antibody specific for soluble serum or plasma BAFF polypeptide as defined elsewhere herein.

In certain embodiments, a sample generating a signal that is statistically stronger than the predetermined cut-off value is considered positive for an infection or disease condition, whereas a sample generating a signal that is statistically weaker than the predetermined cut-off value is considered positive for an impaired immune system. In certain embodiments, the sample generates a signal that is up to about two standard deviations, up to about three standard deviations, up to about five standard deviations, up to about ten standard deviations, up to about twenty standard deviations, up to about thirty standard deviations, up to about forty standard deviations, up to about fifty standard deviations, up to about sixty standard deviations, up to about seventy standard deviations, up to about eighty standard deviations, up to about ninety standard deviations, or up to about hundred standard deviations above the predetermined cut-off. In other embodiments, the sample generates a signal that is up to about two standard deviations, up to about three standard deviations, up to about five standard deviations, up to about ten standard deviations, up to about twenty standard deviations, up to about thirty standard deviations, up to about forty standard deviations, up to about fifty standard deviations, up to about sixty standard deviations, up to about seventy standard deviations, up to about eighty standard deviations, up to about ninety standard deviations, or up to about hundred standard deviations below the predetermined cut-off.

In other embodiments, the cut-off value is determined using a Receiver Operator Curve, according to the method of Sackett et al., Clinical Epidemiology: A Basic Science for Clinical Medicine, Little Brown and Co., 1985, p. 106-7. Briefly, in these embodiments, the cut-off value may be determined from a plot of pairs of true positive rates *(i.e.,* sensitivity) and false positive rates (100%-specificity) that correspond to each possible cut--off value for the diagnostic test result. The cut-off value on the plot that is the closest to the upper left-hand corner *(i.e.,* the value that encloses the largest area) is the most accurate cut-off value, and a sample generating a signal that is higher than the cut-off value determined by this method may be considered positive. Alternatively, the cut-off value may be shifted to the left along the plot, to minimize the false positive rate, or to the right, to minimize the false negative rate. In general, a sample generating a signal that is higher than the cut-off value determined by this method is considered positive for an infection or disease condition, whereas a sample generating a signal that is three standard deviations below the predetermined cut-off value is considered positive for an impaired immune system.

In some embodiments of the invention, the in vitro use involves the use of a BAFF binding agent immobilized on a solid support to bind to and remove the serum or plasma BAFF polypeptide from the remainder of the sample. The bound BAFF polypeptide may then be detected using a detection reagent that contains a reporter group and specifically binds to the binding agent/polypeptide complex. Such detection reagents may comprise, for example, a binding agent that specifically binds to the soluble serum or plasma BAFF polypeptide as defined herein, or an antibody or other agent that specifically binds to the binding agent, such as an anti-immunoglobulin, protein G5 protein A or a lectin. In some embodiments, the BAFF detection reagent, e.g., antibody, is bound to biotin which recognizes and specifically binds a streptavidin or avidin binding agent.

In certain embodiments, the detection is performed in a lateral flow or strip test format, as discussed elsewhere herein, wherein the BAFF binding agent, e.g., antibody, is immobilized on a membrane, such as nitrocellulose. In the lateral flow test, soluble serum or plasma BAFF polypeptides as defined herein within the sample bind to the immobilized binding agent as the sample passes through the membrane. A second, labeled binding agent then binds to the BAFF binding agent-polypeptide complex as a solution containing the second binding agent flows through the membrane. The detection of bound second binding agent may then be performed as described above. In the strip test format, one end of the membrane to which BAFF binding agent is bound is immersed in a solution containing the sample. The sample migrates along the membrane through a region containing second binding agent and to the area of immobilized binding agent. Concentration of second binding agent at the area of immobilized antibody indicates the immune status of a subject.

In several aspects of the disclosure being present only for illustration purposes, similar methods for determining response of the immune status of a subject to treatment is provided. Since serum BAFF polypeptide levels correlate with the immune status, response to treatment or therapy is monitored by comparing soluble BAFF polypeptide levels in a subject's serum (or other biological sample) at different time points during the course of a treatment regimen. Thus, a rapid and reliable method of monitoring immune status of a subject and response to treatment of immune status, using, for *e.g*., a serum or plasma sample obtained from the subject's bloodstream is also disclosed herein. In particular aspects, the method is practiced by ELISA assay, lateral flow assay, or strip test assay using an antibody specific for soluble BAFF polypeptide or a fragment thereof.

The disclosure present for illustration purposes only further provides systems and kits for monitoring immune status of a subject, comprising a reagent suitable for determining levels of BAFF polypeptide or a fragment thereof in a biological sample obtained from the subject, wherein the biological sample is a serum or plasma sample. In some aspects which are just present for illustration purposes only, the kit includes reagents for performing ELISA, lateral flow, or strip test assays such as an antibody specific for soluble BAFF polypeptide or a fragment thereof. Detection systems and kits of the disclosure which are not part of the invention are described in further detail below.

### C. Detection Systems and Kits

In various aspects of the disclosure present for illustration purposes only, detection systems and kits for monitoring immune status of a subject are provided. A detection system may be used in the in vitro use of the present invention for monitoring multiple myeloma of a subject using a serum or plasma sample of a subject. The diagnostic kit of the disclosure herein which is not part of the invention could include the method for the detection of antigen-antibody reaction in addition to the material. The detection method used within the in vitro use of the present invention is preferably selected from the group consisting of flow cytometry, immunohistochemistry, and enzyme-linked immunosorbent assay (ELISA), radioimmunoassay (RIA), enzyme immunoassay (EIA), fluorescence immunoassay (FIA), luminescence immunoassay (LIA), lateral flow assay, and strip assay. The reactivity of the antigen recognition material could be confirmed using device detecting an enzyme reaction, fluorescence, luminescence, or radiation. In some embodiments, monitoring multiple myeloma of a subject can be performed with a flow cytometry kit, immunohistochemistry kit, ELISA kit or lateral flow or strip kit including the anti-BAFF antibody or an antigen binding fragment thereof.

In some embodiments, monitoring of multiple myeloma of a subject can be performed with a flow cytometry kit, immunohistochemistry kit, ELISA kit or lateral flow or strip kit including an antibody that is specific for soluble serum or plasma BAFF polypeptide as defined herein.

In some aspects of the disclosure being present just for illustration purposes only, a kit or system may comprise one or more or all of the following components: 1) one or more standards comprised of one or more of the biomarker(s) of the invention, such as soluble BAFF polypeptide or a fragment thereof; 2) a binding agent, such as an antibody or a plurality of antibodies, that are specific for the biomarker(s) that are to be assayed for using the kit; 3) written instructions; 4) diluents for samples and the standards; 5) a wash buffer; 6) color reagents; 7) stop solution; and 8) a carrier, such as an antibody carrier, for example, a lateral flow device, or a microplate with bound antibody, or polystyrene beads.

In some embodiments, the detection system used in the in vitro use of the present invention to monitor multiple myeloma of a subject is a quantitative ELISA (enzyme-linked immunosorbent assay) that determines the concentration or concentrations of the biomarker or biomarker(s) in accordance with the in vitro use embodied by the invention. The principle of the assay is to use the quantitative sandwich enzyme immunoassay technique wherein a monoclonal or polyclonal antibody selective for a biomarker is pre-coated onto a carrier such as a microplate into its wells. The standards and sample are then pipetted into the wells and any of the biomarker that is present is bound to this immobilized antibody. Next, the wells are washed with washing buffer, and an enzyme-linked monoclonal or polyclonal antibody that is specific for the biomarker is added to the wells. Washing is again performed, and then a substrate solution is added to the wells. Color subsequently develops in proportion to the amount of polypeptide of the invention that is bound in the first step. The color development is stopped using a stop solution, and the intensity of the color is measured by a microplate reader.

In other embodiments, the monitoring of multiple myeloma of a subject in the in vitro use of the present invention may be carried out using, for example, a lateral flow assay. Such lateral flow assays have the potential to be a cost-effective, fast, simple, and sensitive method, for instance for on-site screening assays. The lateral flow assay comprises a carrier that allows a lateral flow to occur wherein either the sample or the detection reagent is displaced form one location on the carrier to another. There are many formats of lateral flow assays suitable for use in the in vitro use embodied by the invention, and the skilled person will readily know how to select and optimize a particular format. An example of a lateral flow test strip also disclosed herein for illustration purposes only comprises, for example, the following components: sample pad; an absorbent pad onto which the test sample is applied; a conjugate or reagent pad that contains antibodies specific to the target analyte and conjugated to colored particles (usually colloidal gold particles, or latex microspheres); a reaction membrane, typically a hydrophobic nitrocellulose or cellulose acetate membrane onto which anti-target analyte antibodies are immobilized in a line across the membrane as a capture zone or test line (a control zone may also be present, containing antibodies specific for the conjugate antibodies); and a wick or waste reservoir, a further absorbent pad designed to draw the sample across the reaction membrane by capillary action and collect it.

There are a number of variations on lateral flow technology. The capture zone on the membrane may contain immobilized antigens or enzymes depending on the target analyte rather than antibodies. It is also possible to apply multiple capture zones to create a multiplex test. For example, in particular embodiments, test strips able to detect soluble serum or plasma BAFF polypeptide as defined herein and separately in the same sample additional biomarkers of multiple myeloma, e.g., D2M, IL-6, C-reactive protein, and serum monoclonal protein are contemplated. Lateral flow immunoassays are simple to use by untrained operators and generally produce a result within 15 minutes. They are very stable and robust, have a long shelf life and do not usually require refrigeration. They are also relatively inexpensive to produce. These features make them ideal for use at the point-of-care and for testing samples in the field, as well as in the laboratory.

While most lateral flow immunoassays are only capable of providing a qualitative result, it is possible to obtain some degree of quantification by measuring the amount of conjugate bound to the capture zone. This can be done using a dedicated reader to measure the intensity of the colored test line. For example, the Neogen Corporation has developed the Accuscan^{™} lateral flow reader for use with its range of Reveal^{®} assay kits and Charm Sciences also supplies a reader for its Rosa^{®} range of test strips. More sophisticated techniques, such as fluorescent dye labeled conjugates, have also been developed to improve the quantitative potential of lateral flow assays.

A detection system in kit form can include, for example, in an amount sufficient for at least one assay a polyclonal antibody composition or a monoclonal antibody composition that binds soluble serum or plasma BAFF polypeptide as defined herein, as a packaged reagent. Instructions for use of the packaged reagent are also typically included.

A detection system in kit form can also include, for example, a means for combining the test sample with a buffering system (Reagent 1) containing viscosity controllers and stabilizers into a reaction vessel and mixing the solution. A detection system in kit form can also include a means for reading the a parameter of the reaction vessel with sample and buffer, and further means for combining the test sample and buffer mixture with a fluorescence-labeled ligand (Reagent 2) to said biological substance in the reaction vessel, mixing the solution to produce an assay solution. Furthermore, Reagent 2 may be delivered to the reaction vessel without further dilution volume of the assay solution.

As used herein, the term "package" refers to a solid matrix or material such as glass, plastic, paper, foil and the like capable of holding within fixed limits an antibody composition or monoclonal antibody composition. Thus, for example, a package can be a glass vial used to contain milligram quantities of a contemplated polypeptide or it can be a microtiter plate well to which microgram quantities of a contemplated polypeptide or antibody have been operatively affixed.

"Instructions for use" typically include a tangible expression describing the reagent concentration or at least one assay method parameter such as the relative amounts of reagent and sample to be admixed, maintenance time periods for reagent/sample admixtures, temperature, buffer conditions and the like.

In particular embodiments, a detection system used in the in vitro use of the present invention further includes a label or indicating means capable of signaling the formation of a complex containing a polypeptide or antibody molecule of the present invention.

"Complex" as used herein refers to the product of a specific binding reaction such as an antibody-antigen or receptor-ligand reaction. Exemplary complexes are immunoreaction products.

As used herein, the terms "label" and "indicating means" in their various grammatical forms refer to single atoms and molecules that are either directly or indirectly involved in the production of a detectable signal to indicate the presence of a complex. Any label or indicating means can be linked to or incorporated in an expressed protein, polypeptide, or antibody molecule that is part of an antibody or monoclonal antibody composition, or used separately, and those atoms or molecules can be used alone or in conjunction with additional reagents such labels are themselves well-known in clinical diagnostic chemistry and constitute a part of this invention only insofar as they are utilized with otherwise novel proteins methods and/or systems.

The labeling means can be a fluorescent labeling agent that chemically binds to antibodies or antigens without denaturing them to form a fluorochrome (dye) that is a useful immunofluorescent tracer. Suitable fluorescent labeling agents are fluorochromes such as fluorescein isocyanate (FIC), fluorescein isothiocyante (FITC), 5-dimethylamine-1-naphthalenesulfonyl chloride (DANSC), tetramethylrhodamine isothiocyanate (TRITC), lissamine, rhodamine 8200 sulphonyl chloride (RB 200 SC) and the like. A description of immunofluorescence analysis techniques is found in DeLuca, "Immunofluorescence Analysis", in Antibody As a Tool, Marchalonis, et al., eds., John Wiley & Sons, Ltd., pp. 189-231 (1982).

In certain embodiments, the indicating group is an enzyme, such as horseradish peroxidase (HRP), glucose oxidase, or the like. In such cases where the principal indicating group is an enzyme such as HRP or glucose oxidase, additional reagents are required to visualize the fact that a receptor-ligand complex (immunoreactant) has formed. Such additional reagents for HRP include hydrogen peroxide and an oxidation dye precursor such as diaminobenzidine. An additional reagent useful with glucose oxidase is 2,2'-azino-di-(3-ethyl-benzthiazoline-G-sulfonic acid) (ABTS).

In other embodiments, the indicating group is a green fluorescent protein (GFP).

Radioactive elements are also useful labeling agents and are used illustratively herein. An exemplary radiolabeling agent is a radioactive element that produces gamma ray emissions. Elements which themselves emit gamma rays, such as ¹²⁴I, ¹²⁵I, ¹²⁸I, ¹³²I and ⁵¹Cr represent one class of gamma ray emission-producing radioactive element indicating groups. Particularly preferred is ¹²⁵I. Another group of useful labeling means are those elements such as ¹¹C, ¹⁸F, ¹⁵O and ¹³N which themselves emit positrons. The positrons so emitted produce gamma rays upon encounters with electrons present in the animal's body. Also useful is a beta emitter, such ¹¹¹indium or ³H.

The linking of labels, *i*.*e*., labeling of, polypeptides and proteins is well known in the art. For instance, antibody molecules produced by a hybridoma can be labeled by metabolic incorporation of radioisotope-containing amino acids provided as a component in the culture medium. *See*, for example, Galfre et al., Meth. Enzymol., 73:3-46 (1981). The techniques of protein conjugation or coupling through activated functional groups are particularly applicable. *See*, for example, Aurameas, et al., Scand. J. Immunol., Vol. 8 Suppl. 7:7-23 (1978), Rodwell et al., Biotech., 3:889-894 (1984), and U.S. Pat. No. 4,493,795.

The detection systems used in the in vitro use of the present invention can be used in an "ELISA" format to detect, for example, the presence or quantity of soluble serum or plasma BAFF polypeptide as defined herein in a plasma or serum sample.. "ELISA" refers to an enzyme-linked immunosorbent assay that employs an antibody or antigen bound to a solid phase and an enzyme-antigen or enzyme-antibody conjugate to detect and quantify the amount of an antigen or antibody present in a sample. Thus, for example, a polypeptide, antibody molecule composition or monoclonal antibody molecule composition of the present invention can be affixed to a solid matrix to form a solid support that comprises a package in the subject diagnostic systems. The reagent is typically affixed to the solid matrix by adsorption from an aqueous medium although other modes of affixation, well known to those skilled in the art, can be used.

Useful solid matrices are also well known in the art. Such materials are water insoluble and include cross-linked dextran; agarose; beads of polystyrene beads about 1 micron to about 5 millimeters in diameter; polyvinyl chloride, polystyrene, cross-linked polyacrylamide, nitrocellulose- or nylon-based webs such as sheets, strips or paddles; or tubes, plates or the wells of a microtiter plate such as those made from polystyrene or polyvinylchloride.

The reagent species, labeled specific binding agent or amplifying reagent of any detection system described herein can be provided in solution, as a liquid dispersion or as a substantially dry power, e.g., in lyophilized form. Where the indicating means is an enzyme, the enzyme's substrate can also be provided in a separate package of a system. A solid support such as the before-described microtiter plate and one or more buffers can also be included as separately packaged elements in this detection assay system.

The packaging materials discussed herein in relation to detection systems are those customarily utilized in diagnostic systems. Such materials include glass and plastic (e.g., polyethylene, polypropylene and polycarbonate) bottles, vials, plastic and plastic-foil laminated envelopes and the like. In some embodiments, a detection system used in the in vitro use of the present invention is useful for assaying for the presence of soluble serum or plasma BAFF polypeptide as defined herein. Such a system comprises, in kit form, a package containing an antibody to soluble serum or plasma BAFF polypeptide as defined elsewhere herein.

### EXAMPLES

### Example 1: BCMA and BAFF Form Complexes In Vitro

100 µL of the anti-mouse BAFF capture antibody was added to 25 µL of undiluted plasma from mice with and without MM xenografts. The mixture was vortexed overnight at room temperature (RT). 100 µL/well of this solution was added to a 96-well microplate, which was rotated on an orbital rotator overnight at RT. The plates were decanted and washed three times with 200 µL wash solution, patted dry and incubated with 200 µL blocking solution for 1 hour at RT. The plates were decanted again and washed X 3 with 200 µL wash solution and patted dry. The anti-human BCMA detection antibody was diluted in the working concentration using phosphate buffered saline (PBS) and 100 µL of this solution added. The plate was rotated overnight at RT, decanted and washed X 3 with 200 µL wash solution and patted dry. Next, the streptavidin was prepared to a working concentration in PBS, and 100 µL added to the wells for 20 minutes at RT in the dark and decanted, and the plate washed X 3 with 200 µL of wash solution, patted dry. Substrate was added at100 µL/well in the dark. After 20 minutes, 50 µL of stop solution was added and absorbance at 450 nm was measured.

Recombinant mouse BAFF (rmBAFF)-recombinant human BCMA (rhBCMA) complexes were detected *in vitro* (Figure 1A). Varying concentrations of 100:1 ratio of rhBCMA:rmBAFF were incubated overnight in a 35°C water bath, and their levels were determined using an ELISA against negative controls of rmBAFF and rhBCMA at high standard concentrations (2 ng/ml) using anti-mBAFF capture and anti-hBCMA detection antibodies. The experiment shows that complexes are detected at ratios and concentrations of BCMA to BAFF that typically are present in MM patients. Mouse BAFF (mBAFF; derived from SCID plasma)-rhBCMA complexes were also detected *in vitro* (Figure 1B). Samples that included CB17 SCID plasma and recombinant human BCMA, and IgG were incubated overnight in a 35°C water bath. Negative controls were determined using an ELISA against rmBAFF and rhBCMA at high standard concentrations (2 ng/ml) using anti-mBAFF capture and anti-hBCMA detection antibodies. All samples were run in triplicates and the average ± SEM was graphed. Absorbance was determined by a µQuant microplate spectrophotometer with KC Junior software.

### Example 2: BCMA and BAFF Complexes Form In Vivo

It was investigated whether BCMA-BAFF complexes form *in vivo.* Plasma samples from C57 Bl/6 mice injected with rhBCMA-Fc were incubated in wells pre-coated with anti-mBAFF capture antibodies. A polyclonal anti-hBCMA detection antibody was then added. A strong absorbance indicating the presence of rhBCMA-mBAFF complexes was observed in samples collected on days 4 and 6 post-rhBCMA injection (Figure 2A). Only minimal background absorbance was observed in samples from mice injected with the control protein Ig-Fc or from untreated control mice, and antibody cross-reactivity was negative.

Similarly, SCID mice were dosed with rhBCMA-Fc at day 0; animals were bled just prior to its administration, and on post-injection days 1, 2 and 7. Plasma from immune deficient SCID mice contained rhBCMA-mBAFF complexes (Figure 2B; top panel), which could still be detected on days 2 and 7. The corresponding mBAFF level following rhBCMA-Fc treatment is shown in (Figure 2B, bottom panel). rhBCMA-mBAFF complexes were also observed on day 1 in plasma samples from immune competent C57 Bl/6 mice following rhBCMA injection (Figure 2C).

### Example 3: Plasma human BCMA levels correlate with human MM tumor volume and BAFF-BCMA complexes are found in SCID mice bearing these tumors

Human BCMA (hBCMA) levels correlated with increases in tumor volume in human MM xenograft models (Figure 3A-C). Notably, mBAFF-hBCMA complexes were detected in plasma samples from mice (measured on day 28 post-tumor implantation) containing the three human MM xenografts (Figure 3D). The hBCMA-mBAFF complex levels in the plasma correlate with the amount of BCMA in the plasma in these 3 different human MM xenograft models (Figure 3A-C). Specifically, LAGκ-2 (Figure 3B) had the highest level of plasma BCMA, followed by LAGλ-1 (Figure 3A), and LAGκ-1A (Figure 3C) had the least amount of plasma BCMA; the hBCMA-mBAFF complexes correlated with the amount of BCMA in the plasma of these mice (Figure 3D).

### Example 4: BAFF levels are significantly lower in SCID mice bearing human MM

It was evaluated whether human B cell maturation antigen (BCMA) produced by MM xenografts affects mouse BAFF plasma levels. Four week-old male CB17 SCID mice were implanted with human MM tumors (LAGκ-2, LAGκ-1A or LAGλ-1 developed from MM patients. Tumors were measured and the formula for an ellipsoid volume was applied (4/3 π × [width/2]² × [length/2]). Tumor-bearing mice were dosed with recombinant human BCMA (rhBCMA) and BCMA-BAFF complexes were analyzed in plasma.

Results showed that SCID mice implanted with the human MM tumor LAGλ-1 had lower mouse BAFF (mBAFF) levels (day 14: *P* = 0.0143; day 21: *P* = 0.0002; and day 28: *P* = 0.0008) when compared with age and sex-matched SCID mice without tumors (Figure 4A). mBAFF was also decreased in plasma collected on days 14, 21, 28, and 35 from mice implanted with LAGκ-2 tumors, compared with mice without tumors (P = 0.0015, *P* < 0.0001, *P* < 0.0001, and *P* < 0.0001, respectively; Figure 4B). Similarly, mice bearing LAGκ-1A xenografts had significantly lower mBAFF levels when compared to mice without tumors.

When comparing two groups, significance was determined using a Student's t-test. The minimal level of significance was *P* < 0.05. Statistical significance of differences observed between two or more groups was determined using ANOVA (two-way). Analysis was determined using GraphPad Prism version 4.03 for Windows, GraphPad Software, San Diego, CA. All *in vivo* experiments contained 4 or 5 mice per treatment group.

Next, mBAFF was incubated with rhBCMA overnight and an mBAFF ELISA was performed. At the higher and clinically relevant concentration of BCMA found in MM patients (100 ng/ml), rhBCMA inhibited the ability of the anti-mBAFF antibody to detect mBAFF (Figure 5A). In contrast, human IgG at an identical concentration to that of rhBCMA did not interfere with mBAFF detection. Similarly, recombinant human BAFF (rhBAFF) was incubated with rhBCMA overnight and an rhBAFF ELISA was performed. At the clinically relevant concentration of BCMA found in MM patients (100 ng/ml), rhBCMA inhibited the ability of the anti-BAFF antibody to detect rhBAFF. Human IgG at an identical concentration to that of rhBCMA did not interfere with rhBAFF detection (Figure 5B). These results indicate that the decrease in mBAFF levels in MM xenografts was due to the formation of BAFF-BCMA complexes.

The spleens and lymph nodes of human MM-bearing and non-bearing SCID mice were analyzed for mBAFF mRNA levels using RT-PCR. No differences in these levels were detected in either the spleen (Figure 5C) or lymph nodes of mice with or without human MM. These results indicate that the lower mBAFF levels in mice containing human MM xenografts were not from changes in the expression of this gene.

### Example 5: Serum BCMA-hBAFF Complexes Are Formed In MM Patient Serum

It was determined if sera from MM patients contained BCMA-hBAFF complexes with a polyclonal anti-human BCMA antibody, using ELISA plates coated with an anti-human BAFF (hBAFF) capture antibody. 25 µL of undiluted plasma from MM patients and healthy donors was obtained and vortexed with hBAFF capture antibody overnight at room temperature (RT). 100 µL/well of this solution was added to a 96-well microplate, which was rotated on an orbital rotator overnight at RT. The plates were decanted and washed three times with 200 µL wash solution, patted dry and incubated with 200 µL blocking solution for 1 hour at RT. The plates were decanted again and washed X 3 with 200 µL wash solution and patted dry. A biotin-labeled anti-human BCMA detection antibody was diluted to the working concentration using phosphate buffered saline (PBS) and 100 µL of this solution added. The plate was rotated overnight at RT, decanted and washed X 3 with 200 µL wash solution and patted dry. Next, 100 µL of streptavidin was added to the wells for 20 minutes at RT in the dark and decanted, and the plate washed X 3 with 200 µL of wash solution, patted dry, and substrate was added at 100 µL/well in the dark. After 20 minutes, 50 µL of stop solution was added and absorbance was measured at 450 nm. A strong absorbance indicating the presence of BCMA-BAFF complexes was detected in MM patients (Figure 6). These results are consistent with the *in vitro* binding experiments described in Example 1 above (Figures 5A & B).

### Example 6: Serum BAFF Levels Are Reduced in MM Patients With Active Disease

Serum from MM patients and healthy donors was obtained and the serum BAFF levels were analyzed. MM patients (N=31) with active disease have significantly decreased serum BAFF levels (median=436.0 pg/mL) compared to serum BAFF levels (median=837.5 pg/mL; P = 0.0004) obtained from healthy donors (Figure 7). These results are consistent with the findings in SCID mice, in which animals bearing human MM showed reduced free plasma BAFF levels (Figure 4A & B).

### Example 7: BAFF Levels Are Increased in MM Patients Who Have Achieved Complete Remission

Serum from patients with multiple myeloma (MM) who have achieved complete remission (CR) and healthy donors was obtained and the serum BAFF levels were analyzed. MM patients (N=15) who are in complete remission (CR) show significantly increased serum BAFF levels (median=1395.0 pg/mL1) compared to serum BAFF levels (median=837.5 pg/mL; P =0.0004) obtained from healthy donors (Figure 8).

### Example 8: Serum BCMA Levels Are Inversely Related to Serum BAFF Levels in MM Patients

Serum from patients with multiple myeloma (MM) who have achieved complete remission (CR) was obtained and serum BCMA levels and serum BAFF levels were analyzed. Serum BCMA levels were found to be inversely related to the serum BAFF levels in these MM patients (Figure 10).

### Example 9: MM patient serum and rhBCMA prevent BAFF from binding to Raji or TB94 B-cells

In order to determine if hBCMA present in MM patient serum or rhBCMA-Fc would block BAFF from binding to the surface of B-cells, Raji B-cells were incubated with serum from a healthy subject (0.02 µg/mL) or a MM patient with PD and serum containing high levels of BCMA (0.75 µg/mL) or in CR with low levels of serum BCMA (0.02 µg/mL) or rhBCMA-Fc (3.0 µg/mL) in the presence of rhBAFF (500 ng/mL).

The Raji and TB94 cells were maintained in RPMI 1640 supplemented with 10% fetal bovine serum, 2 mmol/L 1-glutamine, 100 i/u per mL penicillin, 100 µg/mL streptomycin, and essential amino acids in an atmosphere of 5% carbon dioxide (CO₂) at 37°C. Five hundred ng/mL BAFF-his-Flag-Tag was pre-incubated with 3.0 µg/mL rhBCMA or serum from a healthy subject (BCMA level 0.02 µg/mL) or a MM patient in complete remission (CR) with a similarly low serum BCMA level (0.02 µg/mL) or with PD with a high serum level (0.75 µg/mL) or control mouse IgG in 1 mL 1X PBS at 4°C overnight. Six million Raji or TB94 B-cells were incubated with or without BAFF with or without healthy subject or MM patient serum or rhBCMA for 45 minutes at 37°C. For the blocking experiment, the anti-human BCMA antibody (3.0 µg/mL) was added to rhBCMA or MM serum overnight at 4°C, and this was then cultured with the Raji or TB94 cells. After washing three times, the cells were incubated with either anti-Flag antibody conjugated with FITC or isotype control IgG-FITC for 1 hour at 37°C away from light and then washed three times with PBS. Flow cytometric analysis was performed using a Beckman-Coulter FC500 cytometer with Cytomics CXP software.

Serum from the MM patient with high levels of serum BCMA and rhBCMA-Fc decreased rhBAFF detection on Raji cells from 98.6% to 53.9 % and from 98.6% to 44.2%, respectively (Figure 11A). An anti-hBCMA blocking antibody added to Raji B-cells incubated with BAFF and either rhBCMA or serum from this MM patient increased BAFF binding back up to 87.1% and 77.0%, respectively. In contrast, serum from a healthy subject or MM patient in CR with low levels of serum BCMA (both at 0.02 µg/ml) only minimally decreased BAFF binding which is consistent with the low levels of serum BCMA present in healthy subjects and MM patients in CR. In order to confirm that BCMA (from MM serum or rhBCMA) blocks BAFF from binding to the B-cell surface, and that an anti-BCMA antibody restores this binding, the above experiment was performed using an additional human B-cell line, TB94. Similarly, serum from the MM patient with high levels of serum BCMA and rhBCMA-Fc decreased rhBAFF detection on these cells from 94% to 62.1 % and from 98.6% to 48.7%, respectively (Fig. 11B). An anti-hBCMA blocking antibody added to TB94-cells incubated with BAFF and either rhBCMA or serum from this MM patient increased BAFF binding back up to 84.8.1% and 79.6%, respectively.

## Claims

1. In-vitro use of serum or plasma B-cell activating factor (BAFF) polypeptide for monitoring multiple myeloma of a subject, wherein serum or plasma BAFF polypeptide levels are increased in multiple myeloma patients who have achieved complete remission compared to the levels in healthy control subjects, wherein the BAFF polypeptide comprises an amino acid sequence having at least 75% identity with SEQ ID NO: 1.

2. The in vitro use of claim 1, wherein the BAFF polypeptide comprises an amino acid sequence having at least 80% or at least 90% identity with SEQ ID NO: 1.

3. The in vitro use of claim 1, wherein the BAFF polypeptide is detected using a detection system selected from the group consisting of: an immunohistochemistry, enzyme-linked immunosorbent assay (ELISA), radioimmunoassay (RIA), enzyme immunoassay (EIA), fluorescence immunoassay (FIA), luminescence immunoassay (LIA), lateral flow assay, or strip assay.

4. The in vitro use of claim 1, wherein the detection is performed using an antibody specific for BAFF polypeptide.

5. The in vitro use of claim 4, wherein the antibody specific for BAFF polypeptide is a monoclonal antibody or a polyclonal antibody.

## Patentansprüche

1. In-vitro Verwendung des Serum oder Plasma B-Zell aktivierenden Faktor (BAFF)-Polypeptids zum Überwachen von multiplem Myelom bei einem Subjekt, wobei die Serum oder Plasma BAFF-Polypeptidwerte bei Subjekten mit multiplem Myelom, die eine vollständige Remission erreicht haben, verglichen zu den Werten bei gesunden Kontrollsubjekten erhöht sind, wobei das BAFF-Polypeptid eine Aminosäuresequenz mit mindestens 75% Identität mit SEQ-ID-Nr. 1 umfasst.

2. In-vitro Verwendung nach Anspruch 1, wobei das BAFF-Polypeptid eine Aminosäuresequenz mit mindestens 80% oder mindestens 90% Identität mit SEQ-ID-Nr.1 umfasst.

3. In-vitro Verwendung nach Anspruch 1, wobei das BAFF-Polypeptid nachgewiesen wird unter Verwendung eines Nachweissystems ausgewählt aus der Gruppe bestehend aus: einer Immunohistochemie, einem enzymgekoppelten Immunosorbent-Assay (ELISA), Radioimmunoassay (RIA), Enzymimmunoassay (EIA), Fluoreszenzimmunoassay (FIA), Lumineszenzimmunoassay (LIA), Lateral-Flow-Assay, oder Strip-Assay.

4. In-vitro Verwendung nach Anspruch 1, wobei der Nachweis durchgeführt wird unter Verwendung eines für das BAFF-Polypeptid spezifischen Antikörpers.

5. In-vitro Verwendung nach Anspruch 4, wobei der für das BAFF-Polypeptid spezifische Antikörper ein monoklonaler Antikörper oder ein polyklonaler Antikörper ist.

## Revendications

1. Utilisation in vitro de polypeptide du facteur d'activation des cellules B (BAFF) sérique ou plasmatique pour surveiller le myélome multiple chez un sujet, dans lequel les niveaux de polypeptide du facteur d'activation des cellules B (BAFF) sérique ou plasmatique sont augmentés chez les sujets atteints de myélome multiple qui ont atteint une rémission complète par rapport aux niveaux des sujets témoins sains, dans lequel le polypeptide BAFF comporte une séquence d'acides aminés ayant au moins 75 % d'identité avec la SEQ ID N° 1.

2. Utilisation in vitro selon la revendication 1, dans lequel le polypeptide BAFF comporte une séquence d'acides aminés ayant au moins 80 % ou au moins 90 % d'identité avec la SEQ ID N° 1.

3. Utilisation in vitro selon la revendication 1, dans lequel le polypeptide BAFF est détecté en utilisant un système de détection choisi parmi le groupe constitué: d'une immunohistochimie, d'un essai immuno-absorption par enzyme liée (ELISA), d'un essai radio-immunologique (RIA), d'un essai immunoenzymatique (EIA), d'un essai immunologique par fluorescence (FIA), d'un essai immunologique par luminescence (LIA), d'un essai à flux latéral ou d'un essai en bandelette (strip assay).

4. Utilisation in vitro selon la revendication 1, dans lequel la détection est effectuée en utilisant un anticorps spécifique pour le polypeptide BAFF.

5. Utilisation in vitro selon la revendication 4, dans lequel l'anticorps spécifique pour le polypeptide BAFF est un anticorps monoclonal ou un anticorps polyclonal.


### SEQUENCE LISTING

<110> Berenson, James R.
<120> IMPROVED METHODS FOR MONITORING IMMUNE STATUS OF A SUBJECT
<130> IMBC-009/01WO
<150> 62/337,263 <151> 2016-05-16
<160> 2
<170> PatentIn version 3.5
<210> 1
   <211> 164
   <212> PRT
   <213> Homo sapiens
<400> 1
   Glu Gln Lys Leu Ile Ser Glu Glu Asp Leu Asn Lys Glu Leu Gln Gly
   1               5                   10                  15
   Pro Glu Glu Thr Val Thr Gln Asp Cys Leu Gln Leu Ile Ala Asp Ser
               20                  25                  30
   Glu Thr Pro Thr Ile Gln Lys Gly Ser Tyr Thr Phe Val Pro Trp Leu
           35                  40                  45
   Leu Ser Phe Lys Arg Gly Ser Ala Leu Glu Glu Lys Glu Asn Lys Ile
       50                  55                  60
   Leu Val Lys Glu Thr Gly Tyr Phe Phe Ile Tyr Gly Gln Val Leu Tyr
   65                  70                  75                  80
   Thr Asp Lys Thr Tyr Ala Met Gly His Leu Ile Gln Arg Lys Lys Val
                   85                  90                  95
   His Val Phe Gly Asp Glu Leu Ser Leu Val Thr Leu Phe Arg Cys Ile
               100                 105                 110
   Gln Asn Met Pro Glu Thr Leu Pro Asn Asn Ser Cys Tyr Ser Ala Gly
           115                 120                 125
   Ile Ala Lys Leu Glu Glu Gly Asp Glu Leu Gln Leu Ala Ile Pro Arg
       130                 135                 140
   Glu Asn Ala Gln Ile Ser Leu Asp Gly Asp Val Thr Phe Phe Gly Ala
   145                 150                 155                 160
   Leu Lys Leu Leu
<210> 2
   <211> 285
   <212> PRT
   <213> Homo sapiens
<400> 2
   Met Asp Asp Ser Thr Glu Arg Glu Gln Ser Arg Leu Thr Ser Cys Leu
   1 5 10 15
   Lys Lys Arg Glu Glu Met Lys Leu Lys Glu Cys Val Ser Ile Leu Pro
   20 25 30
   Arg Lys Glu Ser Pro Ser Val Arg Ser Ser Lys Asp Gly Lys Leu Leu
   35 40 45
   Ala Ala Thr Leu Leu Leu Ala Leu Leu Ser Cys Cys Leu Thr Val Val
   50 55 60
   Ser Phe Tyr Gln Val Ala Ala Leu Gln Gly Asp Leu Ala Ser Leu Arg
   65 70 75 80
   Ala Glu Leu Gln Gly His His Ala Glu Lys Leu Pro Ala Gly Ala Gly
   85 90 95
   Ala Pro Lys Ala Gly Leu Glu Glu Ala Pro Ala Val Thr Ala Gly Leu
   100 105 110
   Lys Ile Phe Glu Pro Pro Ala Pro Gly Glu Gly Asn Ser Ser Gln Asn
   115 120 125
   Ser Arg Asn Lys Arg Ala Val Gln Gly Pro Glu Glu Thr Val Thr Gln
   130 135 140
   Asp Cys Leu Gln Leu Ile Ala Asp Ser Glu Thr Pro Thr Ile Gln Lys
   145 150 155 160
   Gly Ser Tyr Thr Phe Val Pro Trp Leu Leu Ser Phe Lys Arg Gly Ser
   165 170 175
   Ala Leu Glu Glu Lys Glu Asn Lys Ile Leu Val Lys Glu Thr Gly Tyr
   180 185 190
   Phe Phe Ile Tyr Gly Gln Val Leu Tyr Thr Asp Lys Thr Tyr Ala Met
   195 200 205
   Gly His Leu Ile Gln Arg Lys Lys Val His Val Phe Gly Asp Glu Leu
           210                 215                 220
   Ser Leu Val Thr Leu Phe Arg Cys Ile Gln Asn Met Pro Glu Thr Leu
   225                 230                 235                 240
   Pro Asn Asn Ser Cys Tyr Ser Ala Gly Ile Ala Lys Leu Glu Glu Gly
                   245                 250                 255
   Asp Glu Leu Gln Leu Ala Ile Pro Arg Glu Asn Ala Gln Ile Ser Leu
               260                 265                 270
   Asp Gly Asp Val Thr Phe Phe Gly Ala Leu Lys Leu Leu
           275                 280                 285